# EUROPÄISCHE PATENTSCHRIFT

(11) **EP 3 808 296 B1**
(45) Veröffentlichungstag und Bekanntmachung des Hinweises auf die Patenterteilung: **22.12.2021**
(21) Anmeldenummer: 19203785.1
(22) Anmeldetag: 17.10.2019
(51) Int. Cl.: A61B 17/86

(54) **VORRICHTUNG ZUR LOKALEN APPLIKATION VON UND/ODER ZUM SPÜLEN MIT FLUIDEN**
DEVICE FOR LOCAL APPLICATION OF AND / OR FOR RINSING WITH FLUIDS
DISPOSITIF D'APPLICATION LOCALE DES FLUIDES ET/OU DE RINÇAGE AUX FLUIDES

(43) Veröffentlichungstag der Anmeldung: 21.04.2021
(73) Patentinhaber: Heraeus Medical GmbH, 61273 Wehrheim (DE)
(72) Erfinder: Vogt, Sebastian, 61273 Wehrheim (DE); Kluge, Thomas, 61273 Wehrheim (DE)
(74) Vertreter: Schultheiss & Sterzel Patentanwälte PartG mbB

(56) Entgegenhaltungen:
- DE-U1- 29 914 192
- US-A1- 2005 059 972

## Beschreibung

Die Erfindung betrifft eine Vorrichtung zur temporären lokalen Applikation von pharmazeutischen Fluiden und/oder zum Spülen mit pharmazeutischen Fluiden.

Gegenstand der Erfindung ist insbesondere eine medizinische Vorrichtung zur temporären, lokalen Applikation von und zum Spülen mit pharmazeutischen Fluiden oder anderen medizinischen Fluiden über einen Zeitraum von einem Tag bis zu mehreren Wochen. Die erfindungsgemäße Vorrichtung ist vor allem für die Prävention und auch zur Behandlung von periprothetischen Infektionen von Schrauben- und Plattenosteosynthesen und zur mechanischen Stabilisierung von Frakturen bestimmt. Die erfindungsgemäße Vorrichtung ist als Applikations- und Spülsystem für eine lokale Applikationen von pharmazeutischen Fluiden im und am Knochengewebe über einen Zeitraum von mehreren Tagen bis Wochen vorgesehen und geeignet.

Die lokale Applikation von pharmazeutischen Wirkstoffen wie Antibiotika ist schon seit Jahrzehnten bekannt und bewährt sich insbesondere bei der Behandlung beziehungsweise der Beruhigung von Infektionen des Knochengewebes. Dabei kann man in nicht-resorbierbare und in resorbierbare beziehungsweise biodegradierbare Wirkstoffträger unterscheiden.

Zur chirurgischen Behandlung von Knochenbrüchen haben sich Schrauben- und Plattenosteosynthesen seit Jahren bewährt. Schrauben- und Plattenosteosynthesen werden bei geschlossenen Frakturen als auch bei offenen Frakturen zur Reposition und zur Stabilisierung der Knochenbrüche eingesetzt. Bei offenen Frakturen kann es naturgemäß zu Kontaminationen des offenliegenden Knochengewebes mit Mikroorganismen kommen. Diese Infektionen stellen sehr schwerwiegende Erkrankungen dar. In seltenen Fällen kann es aber auch bei einer Operation einer geschlossenen Fraktur zur Infektion des Knochengewebes kommen. Die Behandlung dieser infizierten Frakturen ist schwierig und komplex. Es gibt eine Vielzahl von unterschiedlichen Behandlungsoptionen. Eine häufig angewandte Vorgehensweise besteht darin, zuerst eine Entfernung des kompletten Osteosynthesematerials und danach ein Debridement des infizierten Gewebes vorzunehmen. Anschließend werden häufig lokale Antibiotikaträger als Implantate eigesetzt, die nach der Implantation über einen relativ kurzen Zeitraum Gentamicin oder andere Antibiotika freisetzen, wie beispielsweise Gentamicin enthaltende Kollagenschwämme und Polymethylmethacrylatketten. Bei Infektionen mit Gentamicin-resistenten und auch mit multiresistenten Mikroorganismen sind diese Gentamicin-freisetzenden Wirkstoffträger nicht ausreichend wirksam. Problematisch ist weiterhin, dass der frakturierte Knochen auch mechanisch stabilisiert werden muss. Das ist besonders schwierig, wenn auch die Schraubenbohrlöcher von der Infektion betroffen sind. In diesen Fällen wird häufig auf ein Fixateur Externe zurückgegriffen, wobei neue Schrauben außerhalb des infizierten Knochenbereichs zur Befestigung des Fixateur Externe in das Knochengewebe eingesetzt werden. Zusätzliche Bohrungen im Knochen sind dazu notwendig. Alternativ können auch bei größeren infizierten Knochenbereichen Saug-Spüldrainagen verwendet werden. Bei infiziertem Knochengewebe kleiner und mittlerer Größe sind konventionelle Saug-Spüldrainagen mitunter schwierig durchzuführen. Knochenschrauben, die direkt im oder am infizierten Knochengewebe pharmazeutische Wirkstoffe freisetzen könnten und die gleichzeitig anlog zu konventionellen Knochenschrauben eine Stabilisierung des frakturierten Knochens bewirken könnten, wären wünschenswert.

Knochenschrauben mit einem zentralen Kanal im Schraubenkörper und damit verbundenen Fenstern sind unter der Bezeichnung kanellierte und fenestrierte Schrauben bekannt. Bisher werden diese Knochenschrauben im Wesentlichen im Spine-Bereich als sogenannte kanellierte und fenestrierte Pedikelschrauben eingesetzt. Dabei wird durch den Kanal der Schrauben Knochenzementteig in den meist osteoporotischen Wirbelkörper eingespritzt. Es bildet sich meistens ein zur Längsachse der Pedikelschraube koaxialer Zementmantel aus. Der Knochenzement härtet anschließend aus und bildet ein Widerlager für die Pedikelschrauben. Eine Vielzahl von kanellierten und fenestrierten Knochenschrauben wurden vorgeschlagen. Exemplarisch sind dafür die Patenschriften und Offenlegungsschriften DE 35 08 759 A1, RU 2 572 481 C1, RU 2 622 613 C2, US 2001/0021852 A1, US 2005/0015059 A1, US 2012/0029578 A1, US 6 214 012 B1, US 9 326 801 B2, US 8 382 808 B2 und US 8 974 505 B2 genannt.

Eine spezielle kanellierte und fenestrierte Knochenschraube ist in der EP 1 622 529 B1 dargestellt. Dabei wird ein Führungselement temporär in den Kanal der Knochenschraube eingesetzt. Das Führungselement dient dem Einspritzen von Knochenzementteig.

Ein ähnlicher Adapter zum Einschrauben einer kanellierten und fenestrierten Knochenschraube wird in der US 6 048 343 A offenbart. Der Adapter hat einen Längskanal und dichtet mit seiner Außenwand die proximale Bohrung der Knochenschraube ab, so dass Flüssigkeiten in die Knochenschraube durch den Adapter eingepresst werden können. Der Adapter wird nach dem Einpressen der Flüssigkeit in den Knochen entfernt.

Eine in der US 10 188 442 B2 beschriebene Schraube ist für die Tumorbehandlung bestimmt und hat drei oder mehrere Längskanäle im Schraubenkörper, die fenestriert sind. Diese Längskanäle sind zum Anschluss an Katheter vorgesehen. Mit den angeschlossenen Kathetern können flüssige Wirkstoffe in den die Schraube umgebenden Knochen appliziert werden.

Eine mit durchgehenden Längsschlitzen versehene Knochenschraube, bei der auch der Schraubenkopf Längsschlitze aufweist wird mit der EP 2 887 899 B1 vorgeschlagen. Die Längsschlitze des Schraubenkörpers sollen eine bessere Freisetzung von Stoffen, wie Knochenzement, aus der Knochenschraube ermöglichen.

Im Patent EP 0 305 417 B1 wird eine Knochenschraube beschrieben, die kaneliert und fenestriert ist. Diese Knochenschraube wird vakuumdicht im Knochen verschraubt. Es wird ein Saug-Spülsystem mit dieser Knochenschraube beschrieben. Dabei wird bei einer kanellierten und fenestrierten Knochenschraube eine Spülflüssigkeit eingeleitet und an einer zweiten kanellierten und fenestrierten Knochenschraube mit Vakuum die Spülflüssigkeit abgesaugt.

In der US 5 601 559 A1 wird eine kanellierte und fenestrierte Knochenschraube beschrieben, die eine systemischen Applikation von pharmazeutischen Wirkstoffen über das Knochengewebe anstelle von einem venösen Zugang ermöglichen soll.

Eine selbstschneidende Knochenschraube mit Längsschlitzen und einem zentralen Kanal wird in der EP 0 595 782 B1 offenbart. Die Schlitze und der Kanal sollen zuerst das bei dem Einschneiden der Schraube geschnittene Knochenmaterial aufnehmen und dann ein Durchwachsen des Knochengewebes mit der Knochenschraube ermöglichen. Ein Schlitz, in dem mehrere Öffnungen zum inneren Kanal angeordnet sind, erstreckt sich dabei in das Gewinde aber nicht bis zum Schraubenkopf. Der Kanal und die Schlitze werden beim Einschneiden der Knochenschraube durch Knochenmaterial blockiert und können dann nicht mehr zum Leiten eines pharmazeutischen Fluids verwendet werden.

Die Patente US 10 357 298 B2, US 10 349 993 B2 und US 9 616 205 B2 offenbaren Implantate und dabei eine implantierbare Schraube, die einen Längskanal mit Fenestrierungen enthält. Im Längskanal ist unterhalb eines Schraubenkopfes eine sich in distaler Richtung vergrößernde Verjüngung angeordnet. Unterhalb dieser Verjüngung befindet sich ein Reservoir mit nach Außen führenden Kanälen. Der Durchmesser des Kanals im Schraubenkopf ist gleich dem Durchmesser des Reservoirs. Die Länge der vom Reservoir ausgehenden Kanäle ist größer als der Innendurchmesser dieser Kanäle.

Dokument DE29914192U offenbart eine Vorrichtung gemäß der Präambel von Anspruch 1.

Der Erfindung liegt die Aufgabe zugrunde, die Nachteile des Stands der Technik zu überwinden. Insbesondere soll eine Vorrichtung zur lokalen Applikation von und zum Spülen mit pharmazeutischen Fluiden, wie zum Beispiel von antibiotischen Lösungen, bereitgestellt werden, mit der eine lokale und temporäre Abgabe des pharmazeutischen Fluids im Bereich des Knochens ermöglicht wird, wie zum Beispiel in infizierten, mit Knochenschrauben und Osteosyntheseplatten fixierten Knochen. Die Vorrichtung soll auch über längere Zeiträume an einem bestimmten Ort zur wiederholten Abgabe des pharmazeutischen Fluids geeignet sein, ohne dass die Vorrichtung hierfür entfernt werden muss. Die Vorrichtung soll kostengünstig zu fertigen sein. Die Behandlung mit der Vorrichtung soll in deren Verlauf anpassbar sein, so dass auf eine Veränderung der Behandlungssituation oder auf einen ausbleibenden Erfolg reagiert werden kann.

Die Aufgabe der Erfindung besteht somit auch darin, eine Vorrichtung in Form eines lokalen Applikations- und Spülsystems für pharmazeutische Fluide zu entwickeln, das zur Prävention und auch zur Behandlung von periprothetischen Infektionen bei Schrauben- und Plattenosteosynthesen und zur gleichzeitigen Reposition und zur mechanischen Stabilisierung von frakturierten Knochen bestimmt ist. Das zu entwickelnde Applikations- und Spülsystem soll eine lokale Applikation und bevorzugt auch eine Spülung mit pharmazeutischen Fluiden bei zumindest teilweiser Weichteildeckung über einen Zeitraum von mehreren Tagen bis Wochen ermöglichen. Es sollen keine antimikrobiellen Wirkstoffträger in den Teilen der Vorrichtung, insbesondere in den Knochenschrauben der Vorrichtung, selbst enthalten sein. Im Gegensatz dazu sollen beliebige antiseptische oder antibiotische Lösungen im Bereich des zu behandelnden Knochens eines Patienten applizierbar sein. Weiterhin ist es zum Ausschließen von Embolien wichtig, dass sich beim Applizieren von pharmazeutischen Fluiden kein Überdruck im Markraum ausbilden kann. Wünschenswert ist es im Fall von Plattenosteosynthesen, dass die pharmazeutischen Fluide auch die den Knochenschrauben angrenzenden Teile der Osteosyntheseplatten erreichen können. Die Knochenschrauben der Vorrichtung sollen im Wesentlichen den Abmessungen und der Gestalt üblicher Knochenschrauben entsprechen. Die Knochenschrauben der Vorrichtung sollen eine mechanische Fixierung der Frakturenden im Fall der Schraubenosteosynthese und im Fall der Plattenosteosynthese eine normale mechanische Fixierung der Osteosyntheseplatten am Knochengewebe ermöglichen. Die Vorrichtung beziehungsweise das Applikations- und Spülsystem soll so beschaffen sein, das nach Beendigung der Einbringung von pharmazeutischen Fluiden die modifizierten Knochenschrauben in gleicher Weise wie die üblichen Knochenschrauben im Knochengewebe verbleiben können, wenn es medizinisch notwendig ist. Weiterhin wird angestrebt, dass ein Rückfluss der pharmazeutischen Fluide außerhalb des menschlichen Körpers möglich ist. Wichtig ist, dass pharmazeutische Fluide beliebiger Zusammensetzung und bevorzugt auch in veränderlicher Zusammensetzung verwendet werden können.

Die Aufgaben der Erfindung werden gelöst durch eine Vorrichtung zur lokalen Applikation von und/oder zum Spülen mit pharmazeutischen Fluiden, die Vorrichtung aufweisend mindestens eine Knochenschraube, die mindestens eine Knochenschraube aufweisend einen Schraubenkörper, ein Außengewinde und einen proximalen Schraubenkopf, wobei mindestens ein Kanal im Schraubenkörper angeordnet ist, wobei der mindestens eine Kanal flüssigkeitsdurchlässig ist, vom Schraubenkopf ausgeht und in wenigstens eine Fluidaustrittsöffnung im Schraubenkörper mündet, wobei die wenigstens eine Fluidaustrittsöffnung vom Schraubenkopf in distaler Richtung beabstandet ist, wenigstens eine das Außengewinde der Knochenschraube durchbrechende axiale Nut, die sich von der wenigstens einen Fluidaustrittsöffnung im Schraubenkörper bis zum Schraubenkopf erstreckt, wobei ein Nutgrund der wenigstens einen axialen Nut im Schraubenkörper tiefer ist als ein Grund des Außengewindes,
mindestens eine Kappe mit einem Verbindungselement zum lösbaren Verbinden der mindestens einen Kappe mit dem proximalen Schraubenkopf der mindestens einen Knochenschraube, wobei in der mindestens einen Kappe ein flüssigkeitsdurchlässiger Kanal angeordnet ist, wobei der Kanal in der mindestens einen Kappe in den mindestens einen Kanal der mindestens einen Knochenschraube mündet, wenn die mindestens eine Kappe über das Verbindungselement mit der mindestens einen Knochenschraube lösbar verbunden ist, und wobei der Kanal an einer Einlassöffnung in der mindestens einen Kappe beginnt,
mindestens einen Schlauch zur Fluidzuführung, der flüssigkeitsdurchlässig mit der Einlassöffnung an einer der mindestens einen Kappe verbunden oder verbindbar ist, so dass ein pharmazeutisches Fluid mit einem Druck durch den mindestens einen Schlauch zur Fluidzuführung, durch den Kanal der mindestens einen Kappe und durch den mindestens einen Kanal der mindestens einen Knochenschraube aus der wenigstens einen Fluidaustrittsöffnung drückbar ist, wenn der mindestens eine Schlauch zur Fluidzuführung mit der mindestens einen Kappe und die mindestens eine Kappe über das Verbindungselement mit der mindestens einen Knochenschraube verbunden sind.

Die axiale Richtung der Knochenschraube erstreckt sich vom Schraubenkopf zu einem gegenüberliegenden (distalen) Schraubenende (der Spitze) der Knochenschraube. Die Angabe axial bezieht sich dabei auf diese Schraubenachse. Die Schraubenachse ist dabei auch die Achse, um die die mindestens eine Knochenschraube beim Einschrauben oder Ausschrauben gedreht wird. Das Gewinde der Knochenschraube läuft also um diese Schraubenachse herum.

Dass der Nutgrund der Nut tiefer ist als der Grund des Außengewindes, bedeutet, dass die Nut tiefer in den Schraubenkörper eingeschnitten ist als das Außengewinde. Unter einem Nutgrund versteht man die tiefste Stelle einer Nut, sowie man unter einem Gewindegrund die tiefste Stelle eines Gewindes versteht.

Die wenigstens eine Fluidaustrittsöffnung ist also erfindungsgemäß von dem Schraubenkopf beabstandet. Bevorzugt ist die wenigstens eine Fluidaustrittsöffnung im Bereich eines distalen Schraubenendes angeordnet. Dass der mindestens eine Kanal in wenigstens eine Fluidaustrittsöffnung im Bereich eines distalen Schraubenendes mündet, bedeutet, dass die wenigstens eine Fluidaustrittsöffnung dichter am distalen Schraubenende der Knochenschraube angeordnet ist als der Schraubenkopf. Bevorzugt ist die wenigstens eine Fluidaustrittsöffnung dichter an dem distalen Schraubenende der Knochenschraube angeordnet als am Schraubenkopf. Besonders bevorzugt ist die wenigstens eine Fluidaustrittsöffnung innerhalb von 5 mm eines distalen Endes des Außengewindes der Knochenschraube angeordnet.

Unter dem Begriff Knochenschrauben werden alle in der Knochenchirurgie üblichen Schrauben verstanden. Die erfindungsgemäße Vorrichtung beziehungsweise das erfindungsgemäße Applikations- und Spülsystem ist besonders bevorzugt mit KortikalisSchrauben und/oder Spongiosa-Schrauben als die mindestens eine Knochenschraube einsetzbar. Die mindestens eine Knochenschraube ist also vorzugsweise mindestens eine Kortikalis-Schraube und/oder Spongiosa-Schraube.

Die Knochenschrauben können beliebige innere und äußere Antriebe besitzen. Häufig werden in der Chirurgie Torx- und Inbus-Antriebe eingesetzt, die auch erfindungsgemäß bevorzugt sind. Die Antriebe sind in den Schraubenköpfen angeordnet.

Der mindestens eine Schlauch kann grundsätzlich aus einem beliebigen Material aufgebaut werden und kann sogar theoretisch auch aus einem Metall gefertigt sein. Bevorzugt besteht der mindestens eine Schlauch jedoch zumindest bereichsweise aus einem biokompatiblen Kunststoff und ist axial verformbar. Die Verformbarkeit des mindestens einen Schlauchs bewirkt, dass die Behandlungssituation mechanisch weniger stark beansprucht wird.

Bevorzugt enthält die mindestens eine Knochenschraube ein röntgenopakes Material oder besteht aus einem röntgenopaken Material. Hierdurch kann die Position und die Lage der mindestens einen Knochenschraube im Körper des Patienten mit Röntgenverfahren bestimmt werden. Das röntgenopake Material kann besonders bevorzugt ausgewählt sein aus Edelstahl, Titan, Titanlegierungen, Tantal, Tantallegierungen, Bariumsulfat, Bariumsulfat enthaltende Kunststoffe, Zirkoniumdioxid und Zirkoniumdioxid enthaltende Kunststoffe.

Die erfindungsgemäße Vorrichtung ist bevorzugt eine medizinische Vorrichtung.

Vorzugsweise kann die mindestens eine Kappe mit dem Verbindungselement flüssigkeitsdicht oder mit nur einer im Vergleich zum maximal möglichen Volumenstrom durch die mindestens Knochenschraube geringen Leckrate lösbar mit dem proximalen Schraubenkopf der mindestens einen Knochenschraube werden. Die Leckrate ist dann als gering anzusehen, wenn maximal 10% des Volumenstroms durch den mindestens einen Kanal in der mindestens einen Knochenschraube zwischen dem Verbindungselement der mindestens einen Kappe und dem Schraubenkopf der mindestens einen Knochenschraube austritt, wobei bevorzugt maximal 1% des Volumenstroms dort austritt. Hierzu wird angenommen, dass hinter der wenigstens einen Fluidaustrittsöffnung keine weiteren Strömungswiderstände auftreten. Experimentell kann die Leckrate also dadurch bestimmt werden, dass durch eine Kappe eine Flüssigkeit zugeführt wird und in eine mit der Kappe verbundene Knochenschraube geleitet wird und der Volumenstrom, der zwischen der Kappe und dem Schraubenkopf austritt als Leckstrom verglichen wird mit dem Volumenstrom, der aus der wenigstens einen Fluidaustrittsöffnung austritt.

Ein pharmazeutisches Fluid enthält wenigstens einen pharmazeutischen Wirkstoff. Besonders bevorzugt werden Lösungen enthaltend wenigstens ein Antibiotikum, wenigstens ein Zytostatikum, wenigstens ein Chemotherapeutikum und/oder wenigstens ein Antimykotikum. Alternative pharmazeutische Fluide können desinfizierende Bestandteile enthalten. Unter dem Begriff "pharmazeutisches Fluid" werden demzufolge wässrige und auch nichtwässrige Lösungen und Suspensionen von pharmazeutischen Wirkstoffen verstanden. Weiterhin werden unter dem Begriff "pharmazeutisches Fluid" auch Gemische und Lösungen von Gasen in Wasser, Wasser enthaltenden Flüssigkeiten und nichtwässrigen Flüssigkeiten verstanden. Der Begriff "pharmazeutisches Fluid" umfasst bevorzugt auch Gase und Gasgemische.

Es kann ferner vorgesehen sein, dass im Schlauch für die Fluidzuleitung oder in der Verbindung zu einem Behälter für das pharmazeutische Fluid ein Ventilelement, insbesondere ein Rückschlagventil, angeordnet ist, das eine Strömung des pharmazeutischen Fluids aus dem Schlauch in Richtung des Behälters verhindert.

Hierdurch wird sichergestellt, dass kein kontaminiertes pharmazeutisches Fluid aus dem Schlauch in den Behälter für das pharmazeutische Fluid vordringen kann.

Bei erfindungsgemäßen Vorrichtungen kann auch vorgesehen sein, dass die mindestens eine Knochenschraube wenigstens eine radiale Nut aufweist, die in der Oberfläche des Schraubenkopfs der mindestens einen Knochenschraube angeordnet ist und die mit der wenigstens einen axialen Nut im Schraubenkörper verbunden ist.

Hierdurch kann zwischen dem Schraubenkopf der Knochenschraube und einer mit der Knochenschraube fixierten Osteosyntheseplatte ein Kanal für das pharmazeutische Fluid gebildet werden. Damit kann ein Kreislauf zum Durchspülen mit dem pharmazeutischen Fluid bereitgestellt werden, der bereichsweise über die Schraubenbohrung der Knochenschraube im Knochen verläuft und so zur direkten medizinischen Behandlung eingesetzt werden kann. Durch die wenigstens eine radiale Nut kann das pharmazeutische Fluid problemlos am Schraubenkopf vorbeifließen, wenn der Schraubenkopf eine Osteosyntheseplatte fixiert. Die radiale Nut kann zusätzlich zu einer radialen Erstreckung auch eine axiale Erstreckung aufweisen. Innerhalb des Knochens kann sich so kein Druck des pharmazeutischen Fluids aufbauen, der auf die Behandlungssituation drückt.

Ferner kann auch vorgesehen sein, dass sich die wenigstens eine das Außengewinde der Knochenschraube durchbrechende axiale Nut von der wenigstens einen Fluidaustrittsöffnung am distalen Schraubenkörper bis zu einer distalen Seite des Schraubenkopfs erstreckt, wobei bevorzugt sich die wenigstens eine axiale Nut bis auf die radial außen liegende Seite des Schraubenkopfs erstreckt.

Auch diese Ausführung dient der Ausbildung eines umlaufenden Kanals für das pharmazeutische Fluid zum Zweck der Erzeugung eines Spülkreislaufs des pharmazeutischen Fluids und/oder zur Vermeidung des Abbauens eines statischen Drucks des pharmazeutischen Fluids auf den zu behandelnden Knochen.

Des Weiteren kann vorgesehen sein, dass das Verbindungselement ein Vorsprung an einer zum Schraubenkopf weisenden Kappenunterseite ist, wobei der Vorsprung den für Flüssigkeiten durchlässigen Kanal in der mindestens einen Kappe umfasst, wobei die mindestens eine Kappe mit dem Vorsprung in den mindestens einen Kanal der mindestens einen Knochenschraube reversibel eingesteckt ist oder einsteckbar ist, wobei bevorzugt der Vorsprung im Zentrum der Kappenunterseite angeordnet ist, besonders bevorzugt axial entlang einer Symmetrieachse der mindestens einen Kappe angeordnet ist.

Hierdurch kann das Verbindungselement sehr leicht und auf einfache Weise mit der mindestens einen Knochenschraube verbunden werden. Zudem kann so ohne großen Aufwand ein flüssigkeitsleitender Kanal durch die mindestens eine Kappe und die mindestens eine Knochenschraube bereitgestellt werden.

Die Richtungsbezeichnung "an einer zum Schraubenkopf weisenden Kappenunterseite der mindestens einen Kappe", bezieht sich auf den Zustand, bei dem die mindestens eine Kappe mit dem Verbindungselement mit der mindestens einen Knochenschraube lösbar verbunden ist.

Dabei kann vorgesehen sein, dass die mindestens eine Kappe an der zum Schraubenkopf weisenden Kappenunterseite zumindest eine sich in radialer Richtung von dem Vorsprung nach außen erstreckende Nut aufweist, wobei die zumindest eine Nut der mindestens einen Kappe sich bevorzugt bis zum radialen Rand der mindestens einen Kappe erstreckt und/oder ein Schlauch zur Fluidableitung mit der zumindest einen Nut der mindestens einen Kappe flüssigkeitsdurchlässig verbunden ist.

Die zumindest eine Nut an der Kappenunterseite kann zum Ableiten des pharmazeutischen Fluids verwendet werden. Dadurch kann ein Kreislauf des pharmazeutischen Fluids bereitgestellt werden oder zumindest das pharmazeutische Fluid von dem zu behandelnden Knochen weggeleitet werden.

Es kann auch vorgesehen sein, dass der mindestens eine Schlauch zur Fluidzuführung mit einem Fluidreservoir flüssigkeitsdurchlässig verbunden oder verbindbar ist, wobei ein pharmazeutisches Fluid aus dem Fluidreservoir unter Druck in den mindestens einen Schlauch zur Fluidzuführung durch den Kanal der mindestens einen Kappe und den mindestens einen Kanal der mindestens einen Knochenschraube pressbar ist.

Hierdurch wird die Vorrichtung weiter komplettiert und ist unmittelbar zur Behandlung einsetzbar.

Erfindungsgemäß kann auch vorgesehen sein, dass der mindestens eine Kanal im Schraubenkörper außer an der wenigstens einen Fluidaustrittsöffnung nicht mit der wenigstens einen axialen Nut im Schraubenkörper verbunden ist.

Hierdurch wird sichergestellt, dass das pharmazeutische Fluid an der wenigstens einen Fluidaustrittsöffnung an dem distalen Schraubenende austritt und somit dort in vollem Umfang zur Behandlung zur Verfügung steht. Insbesondere erfolgt eine Spülung mit dem pharmazeutischen Fluid auch im Bereich der wenigstens einen Fluidaustrittsöffnung an dem distalen Schraubenende.

Des Weiteren kann vorgesehen sein, dass die mindestens eine Kappe kuppelförmig mit einer bis auf das Verbindungselement planen Unterseite ausgebildet ist und/oder die mindestens eine Kappe den Schraubenkopf vollständig abdeckt, bevorzugt die mindestens eine Kappe den Schraubenkopf überlappt.

Es kann auch vorgesehen sein, dass die mindestens eine Kappe einen zumindest genauso großen Durchmesser aufweist, wie der äußere Durchmesser des Schraubenkopfs.

Diese Maßnahmen dienen dazu, eine möglichst vollflächige und gut dichtbare Verbindung zwischen der mindestens eine Kappe und dem Schraubenkopf der mindestens einen Knochenschraube zu ermöglichen. Durch die kuppelförmige Gestalt wird sichergestellt, dass es nicht durch scharfe Kanten zu Verletzungen des überdeckenden Weichgewebes kommt. Durch die Überdeckung der mindestens einen Knochenschraube mit der mindestens einen Kappe kann die jeweilige Kappe so groß ausgebildet werden, dass die Kappe nur flach ansteigende Kanten hat, wodurch das überdeckende Weichgewebe geschont wird.

Bevorzugt kann auch vorgesehen sein, dass die mindestens eine Kappe eine Öffnung oder mehrere Öffnungen zum Ableiten von Fluiden aufweist, die mit einem Schlauch zur Fluidableitung verbindbar oder verbunden sind, wobei die Öffnung oder die Öffnungen vorzugsweise an einer zum Schraubenkopf weisenden Kappenunterseite der mindestens einen Kappe angeordnet ist oder sind.

Hierdurch kann die mindestens eine Kappe zum Erzeugen des Kreislaufs des pharmazeutischen Fluids genutzt werden. Durch die Öffnung oder die mehreren Öffnungen zum Ableiten von Fluiden können die pharmazeutischen Fluide mit einem Schlauch zum Ableiten des pharmazeutischen Fluids aus dem Bereich des Schraubenkopfs ausgeleitet werden. Eine unerwünschte lokale Ansammlung von pharmazeutischem Fluid oberhalb und neben der Kappe wird dadurch wirksam verhindert. Mit dieser Ausgestaltungsvariante ist eine kontinuierliche Spülung des Bohrkanals der Knochenschraube und des angrenzenden Knochengewebes mit pharmazeutischen Fluiden, speziell mit antibiotischen Lösungen möglich. Durch die Anordnung der Öffnung oder der mehreren Öffnungen an der Kappenunterseite der mindestens einen Kappe werden die pharmazeutischen Fluide direkt nach ihrem Austritt am Schraubenkopf abgeleitet.

Dabei kann vorgesehen sein, dass der mindestens eine Schlauch zur Fluidzuführung und der Schlauch zur Fluidableitung in Längsrichtung miteinander verbunden sind, bevorzugt parallel nebeneinander miteinander verbunden sind oder koaxial zueinander angeordnet sind.

Hierdurch wird die Handhabung vereinfacht. Zudem sind dadurch eine platzsparende Einleitung und Ausleitung von pharmazeutischen Fluiden möglich.

Gemäß einer weitere Ausgestaltung der vorliegenden Erfindung kann vorgesehen sein, dass die Vorrichtung zumindest eine Osteosyntheseplatte aufweist. Auch kann vorgesehen sein, dass die mindestens eine Kappe wenigstens ein Rastelement aufweist, mit der die mindestens eine Kappe mit einer Osteosyntheseplatte zu verrasten ist, wobei bevorzugt das wenigstens eine Rastelement an einer zum Schraubenkopf weisenden Kappenunterseite der mindestens einen Kappe angeordnet ist.

Hierdurch kann die mindestens Kappe zum Sichern der Knochenschraube an der Osteosyntheseplatte verwendet werden. Durch das Rastelement kann die mindestens eine Kappe reversibel an der Osteosyntheseplatte befestigt werden, um ein unbeabsichtigtes Herausrutschen des Verbindungselements aus dem mindestens einen Kanal der mindestens einen Knochenschraube zu verhindern.

Es kann erfindungsgemäß auch vorgesehen sein, dass die Vorrichtung zumindest eine Osteosyntheseplatte aufweist. Dabei kann vorgesehen sein, dass die Osteosyntheseplatte mehrere Löcher aufweist, vorzugsweise mehrere Löcher mit einem Durchmesser aufweist, der kleiner ist als der Schraubenkopf der wenigstens einen Knochenschraube und der größer ist als der Außendurchmesser des Außengewindes der wenigstens einen Knochenschraube. Hierdurch kann die Vorrichtung auch zum Fixieren einer Fraktur verwendet werden.

Ferner kann vorgesehen sein, dass der mindestens eine Schlauch zur Fluidzuführung eine maximale radiale Dehnung von 5 Prozent, insbesondere eine maximale radiale Dehnung von 1 Prozent, bei einem Innendruck von 5 bar aufweist, wobei bevorzugt auch ein Schlauch zur Fluidableitung eine maximale radiale Dehnung von 5 Prozent, insbesondere eine maximale radiale Dehnung von 1 Prozent, bei einem Innendruck von 5 bar aufweist.

Hierdurch wird sichergestellt, dass der Schlauch oder die Schläuche sich beim Einpressen des pharmazeutischen Fluids nicht zu sehr ausdehnen und anschließend einen elastischen Druck über das pharmazeutische Fluid darin auf den zu behandelnden Knochen ausüben. Zudem wird dadurch ein Nachströmen oder Rückströmen des pharmazeutischen Fluids vermieden, wenn der Druck zum Antreiben des Fluidstroms reduziert oder zurückgenommen wird. Durch diese Maßnahmen kann verhindert werden, dass sich beim Applizieren von pharmazeutischen Fluiden ein Überdruck im Markraum ausbilden kann, der zu Embolien führen könnte. Dadurch wird ferner gewährleistet, dass bei dem Einpressen von pharmazeutischen Fluiden in den mindestens einen Schlauch zur Fluidzuführung und gegebenenfalls auch beim Fluidrückfluss durch den Schlauch zur Fluidableitung praktisch keine radiale Dehnung der Schläuche erfolgt. Dadurch können Schmerzen am infizierten beziehungsweise am entzündeten Gewebe während der Applikation der pharmazeutischen Fluide vermieden werden.

Des Weiteren kann vorgesehen sein, dass die mindestens eine Kappe mindestens zwei Kappen sind und die mindestens eine Knochenschraube mindestens zwei Knochenschrauben sind, wobei die mindestens zwei Kappen mit ihren Verbindungselementen in zwei unterschiedliche der mindestens zwei Knochenschrauben befestigt sind, wobei die mindestens zwei Kappen durch ein Rohr oder zwei Rohre flüssigkeitsdurchlässig miteinander verbunden sind, wobei der mindestens eine Schlauch zur Fluidzuführung nur mit einer der mindestens zwei Kappen flüssigkeitsdurchlässig verbunden ist, wobei das pharmazeutische Fluid über die durch das eine Rohr oder die zwei Rohre miteinander verbundenen zumindest zwei Kappen auf die zumindest zwei Knochenschrauben verteilbar ist oder wobei der mindestens eine Schlauch zur Fluidzuführung und ein Schlauch zur Fluidableitung nur mit einer der mindestens zwei Kappen flüssigkeitsdurchlässig verbunden sind, wobei das pharmazeutische Fluid über die durch das eine Rohr oder die zwei Rohre miteinander verbundenen zumindest zwei Kappen seriell durch die zumindest zwei Knochenschrauben leitbar ist.

Hierdurch können mehrere Knochenschrauben über einen gemeinsamen Anschluss gemeinsam zum Spülen mit dem pharmazeutischen Fluid verwendet werden.

Ebenso ist es möglich, koaxiale Rohre zu verwenden, so dass eine Fluidabführung über nur eine der mindestens zwei Kappen und einen damit verbundenen Schlauch zum Ableiten des pharmazeutischen Fluids erfolgen kann. Das Rohr oder die Rohre können teleskopartig gegeneinander verschiebbar ineinandergesteckt werden. Ebenso ist es möglich, die mindestens zwei Kappen und das Rohr oder die Rohre in einem flachen streifenförmigen Kunststoffkörper unterzubringen.

Erfindungsgemäß kann vorgesehen sein, dass in den mindestens zwei Knochenschrauben jeweils eine der mindestens zwei Kappen eingesteckt ist, wobei die Kappen durch zwei elastische Rohre flüssigkeitsdurchlässig miteinander verbunden sind, wobei der Schlauch zum Zuführen des pharmazeutischen Fluids und der Schlauch zum Ableiten des pharmazeutischen Fluids mit nur mit jeweils einer Kappe flüssigkeitsdurchlässig verbunden sind, so dass pharmazeutische Fluide über die miteinander verbundenen Kappen auf die mindestens zwei Knochenschrauben verteilbar ist und das rückfließende pharmazeutische Fluid über den Schlauch zum Ableiten des pharmazeutischen Fluids abzuleiten ist.

Es kann auch vorgesehen sein, dass die Vorrichtung einen Behälter für das pharmazeutische Fluid aufweist, wobei bevorzugt der Behälter einen Hohlzylinder mit einem axial im Hohlzylinder verschiebbaren Kolben aufweist, der ein erstes Ende des Hohlzylinders verschließt, wobei der Hohlzylinder an einem dem ersten Ende gegenüberliegenden Ende eine Austragsöffnung aufweist, die mit dem mindestens einen Schlauch zur Fluidzuführung verbunden oder verbindbar ist, besonders bevorzugt über ein manuell bedienbares Ventilelement zur Regulierung der Strömungsgeschwindigkeit des pharmazeutischen Fluids mit dem mindestens einen Schlauch zur Fluidzuführung verbunden oder verbindbar ist.

Hierdurch kann die Vorrichtung auch gleichzeitig zum Erzeugen eines Volumenstroms des pharmazeutischen Fluids genutzt werden. Ferner muss kein separates Reservoir für die pharmazeutische Flüssigkeit an der Vorrichtung angeschlossen werden. Bevorzugt ist der Kolben mit zumindest einer gespannten elastischen Feder antreibbar.

Ferner kann vorgesehen sein, dass in dem Behälter ein pharmazeutisches Fluid enthalten ist, insbesondere enthaltend zumindest einen antibiotischen Wirkstoff, zumindest einen antimykotischen Wirkstoff und/oder zumindest ein Chemotherapeutikum.

Hierdurch kann die Vorrichtung unmittelbar zu einer Behandlung eingesetzt werden.

Gemäß einer bevorzugten Weiterbildung der vorliegenden Erfindung kann vorgesehen sein, dass die Vorrichtung eine Fördereinrichtung aufweist, mit der das pharmazeutische Fluid aus einem mit der Fördereinrichtung verbundenen oder verbindbaren Behälter in den mindestens einen Schlauch zur Fluidzuführung, durch den Kanal in der mindestens einen Kappe, durch den mindestens einen Kanal im Schraubenkörper der mindestens einen Knochenschraube und durch die wenigstens eine Fluidaustrittsöffnung in die Umgebung der mindestens einen Knochenschraube drückbar ist, wobei vorzugsweise die Fördereinrichtung ein Energiespeicherelement, insbesondere zumindest eine gespannte Feder, aufweist, wobei die Fördereinrichtung mit Energie aus dem Energiespeicherelement antreibbar ist, wobei besonders bevorzugt mit dem Energiespeicherelement ein Kolben in einem Hohlzylinder als der Behälter in Richtung einer gegenüberliegenden Austragsöffnung anzutreiben ist.

Mit einer Fördereinrichtung kann die Vorrichtung direkt zum Erzeugen eines Volumenstroms des pharmazeutischen Fluids verwendet werden. Wenn die Fördereinrichtung ein Energiespeicherelement umfasst, muss die Vorrichtung nicht an eine externe Energieversorgung zum Antreiben der Fördereinrichtung angeschlossen werden. Eine gespannte Feder enthält dabei genug Energie, um eine Menge von einigen Millilitern bis einigen Zentilitern des pharmazeutischen Fluids mit der Vorrichtung auszupressen.

Des Weiteren kann vorgesehen sein, dass die Vorrichtung zumindest eine Verschlusskappe aufweist, mit der der mindestens eine Kanal im Schraubenkörper der Knochenschraube reversibel verschließbar ist, wobei vorzugsweise die zumindest eine Verschlusskappe einen proximalen Stift aufweist, mit dem der mindestens eine Kanal bis zur wenigstens einen Fluidaustrittsöffnung reversibel verschließbar ist.

Es ist vorteilhaft, wenn vor und während der Implantation der mindestens eine Kanal der Knochenschraube und ein Hohlraum im Schraubenkopf (als Teilstück des mindestens einen Kanals im Schraubenkörper) durch eine Verschlusskappe umfassend einen manuell zu entfernenden Stift ausgefüllt ist.

Durch solche Maßnahmen wird verhindert, dass der mindestens eine Kanal in der Knochenschraube durch Partikel, wie Knochenspäne, zugesetzt wird. Nach dem Einschrauben der Knochenschraube wird der Stift herausgezogen und der mindestens eine Kanal in der Knochenschraube freigelegt.

Der die Oberkante überragende Teil des Verbindungselements der mindestens einen Verschlusskappe kann bevorzugt als Außensechskant ausgebildet sein. Dadurch ist es möglich mit einem Außensechskant (Inbus) die mindestens eine Knochenschraube in das Bohrloch einzuschrauben. Voraussetzung ist dafür, dass das Verbindungselement und der Außensechskant aus einem mechanisch stabilen Material, wie zum Beispiel Stahl, gefertigt ist, damit beim Einschrauben keine Torsion des Verbindungselements und des Sechskants auftritt.

Ferner kann erfindungsgemäß vorgesehen sein, dass der die Oberkante überragende Teil der mindestens einen Verschlusskappe als Handgriff ausgebildet ist. Dadurch ist es möglich, mit dem Handgriff und dem Verbindungsmittel die Knochenschrauben in das vorgebohrte Bohrloch einzuschrauben. Voraussetzung ist dafür jedoch, dass das Verbindungsmittel und der Handgriff entweder aus hochfestem Kunststoff oder aus Metall gefertigt sind, um ausreichend torsionsstabil zu sein.

Der Erfindung liegt die überraschende Erkenntnis zugrunde, dass es durch die Kanäle in der mindestens einen Knochenschraube und in der mindestens einen Kappe sowie durch die mindestens eine axiale Nut gelingt, die Umgebung der Knochenschraube und damit den mit der Vorrichtung zu behandelnden Knochen mit einem pharmazeutischen Fluid zu beaufschlagen beziehungsweise zu bespülen oder zu umspülen und damit für eine anpassbare Behandlung zugänglich zu machen. Durch eine Weiterführung der axialen Nut bis an den radialen Rand des Schraubenkopfs werden auch die Oberflächen des Knochens und der Osteosynthesevorrichtung für das pharmazeutische Fluid zugänglich.

Das erfindungsgemäße Vorrichtung ermöglicht als Applikations- und Spülsystem eine lokale Applikation und Spülung mit pharmazeutischen Fluiden bei zumindest teilweiser Weichteildeckung über einen Zeitraum von mehreren Tagen bis Wochen. Hierfür wurden im Rahmen der vorliegenden Erfindung übliche Knochenschrauben, wie Spongiosa- und Kortikalisschrauben, so modifiziert, dass pharmazeutische Fluide, wie wässrige antiseptische oder antibiotische Lösungen, über die Knochenschrauben bis in die Medulla eingebracht werden können und dass gleichzeitig auch die Bohrkanäle der Knochenschrauben sowie die Außenseite der Knochenschrauben mit den pharmazeutischen Fluiden zu mindestens teilweise umspült werden können.

Die Vorrichtung beziehungsweise das Applikations- und Spülsystem ist so beschaffen, das nach Beendigung der Einbringung von pharmazeutischen Fluiden die modifizierten Knochenschrauben in gleicher Weise wie die üblichen Knochenschrauben im Knochengewebe verbleiben können, wenn es medizinisch notwendig ist. Dazu sind die Teile der Vorrichtung, die der Einleitung der pharmazeutischen Fluide dienen, in einfacher Weise von den Knochenschrauben entfernbar.

Mit dem erfindungsgemäßen Applikationssystem beziehungsweise mit der erfindungsgemäßen Vorrichtung können nach dem Einschrauben der mindestens einen Knochenschraube in das Knochengewebe pharmazeutische Fluide durch den Schlauch und durch die mindestens eine Kappe in den mindestens einen Kanal der Knochenschraube eingeleitet werden. Das pharmazeutische Fluid fließt dann im Bereich des distalen Endes der Knochenschraube aus der wenigstens einen Fluidaustrittsöffnung und kann somit das umliegende Knochengewebe erreichen. Wenn ausreichend Fluid aus der wenigstens einen Fluidaustrittsöffnung nachfließt, migriert das pharmazeutische Fluid dann über die wenigstens eine axiale Nut in Richtung des Schraubenkopfs. Dadurch, dass die axiale Nut in Richtung der Schraubenlängsachse tiefer liegt als der Grund des Gewindes der Knochenschraube, kann ein Fließen in Richtung Schraubenkopf garantiert werden. Das angrenzende Knochengewebe kann den Nutgrund so nicht verschließen. Das umliegende Knochengewebe wird dadurch von dem pharmazeutischen Fluid benetzt. Das pharmazeutische Fluid kann danach durch die radialen Nuten an der Unterseite des Schraubenkopfes vorbei aus dem Bohrkanal im Knochen austreten. Die Ausbildung eines Überdrucks durch das eingepresste Fluid im Knochengewebe, speziell in der Medula, wird dadurch wirksam verhindert. Das pharmazeutische Fluid kann im angrenzenden Knochengewebe als auch auf der Oberfläche der Knochenschraube wirksam werden.

Der besondere Vorteil der erfindungsgemäßen Applikations- und Spülvorrichtung ist, dass eine lokale Applikation von pharmazeutischen Fluiden, besonders von beliebig zusammengesetzten antibiotischen Lösungen, bei gleichzeitiger Stabilisierung des frakturierten Knochens vorgenommen werden kann. Bohrungen im Knochengewebe für zusätzliche Schrauben oder Pins, wie im Fall der Anwendung vom Fixateur Externe, sind nicht notwendig. Eine Applikation von pharmazeutischen Fluiden ist über Stunden bis zu mehreren Tagen möglich. Nach Beendigung der Fluidapplikation wird nur die mindestens eine Kappe mit dem Schlauch von der mindestens einen Knochenschraube entfernt beziehungsweise abgezogen. Die mindestens eine Knochenschraube kann dann weiter zur Frakturstabilsierung im Knochengewebe verbleiben.

Eine beispielhafte erfindungsgemäße Vorrichtung zur lokalen Applikation und/oder zum Spülen für pharmazeutische Fluide ist zusammengesetzt aus
a) mindestens einer Knochenschraube mit einem Außengewinde und einem Schraubenkopf, mit mindestens einem axialen flüssigkeitsdurchlässigen Kanal im Schraubenkörper, der vom Schraubenkopf ausgeht, und mit mindestens einer mit dem Kanal verbundenen Fluidaustrittsöffnung am distalen Schraubenende,
b) mindestens eine das Außengewinde der Knochenschraube durchbrechende axiale Nut, die sich von der Fluidaustrittsöffnung am äußeren Schraubenkörper bis zur Unterseite des Schraubenkopfs erstreckt, wobei der Nutgrund tiefer in Richtung der Längsachse ausgebildet ist, als der Grund des Gewindes,
c) einem Schraubenkopf, der an seiner Unterseite ein oder mehrere radiale Nuten besitzt, die in Verbindung mit der mindestens einen axialen Nut des Schraubenkörpers stehen,
d) einer Kappe mit einem Stift (als Verbindungselement) an der Kappenunterseite, wobei der Stift einen für Flüssigkeiten durchlässigen Kanal besitzt, der mit einem Kanal in der Kappe flüssigkeitsdurchlässig verbunden oder verbindbar ist, der an einer Außenseite der Kappe in eine Einlassöffnung mündet, wobei die Kappe mit dem Stift in den Kanal der Knochenschraube reversibel eingesteckt oder einsteckbar ist, und
e) einem ersten Schlauch zur Fluidzuführung der flüssigkeitsdurchlässig mit der Einlassöffnung der Kappe verbunden ist, und wobei der Schlauch mit einem Fluidreservoir flüssigkeitsdurchlässig verbindbar ist, mit dem das Fluid unter Druck in den Schlauch durch den Kanal der Kappe und den Kanal der Knochenschraube gepresst werden kann.

Beispielhaft ist auch eine Vorrichtung zum Auspressen von pharmazeutischen Fluiden mit einem lokalen Applikations- und Spülsystem erfindungsgemäß, die zusammengesetzt ist aus
a) mindestens einer Knochenschraube mit einem Außengewinde und einem Schraubenkopf, mit mindestens einem axialen flüssigkeitsdurchlässigen Kanal im Schraubenkörper, der vom Schraubenkopf ausgeht, und mit mindestens einer mit dem Kanal verbundenen Fluidaustrittsöffnung am distalen Schraubenende,
b) mindestens eine das Außengewinde der Knochenschraube durchbrechende axiale Nut, die sich von der Fluidaustrittsöffnung am äußeren Schraubenkörper bis zur Unterseite des Schraubenkopfs erstreckt, wobei der Nutgrund tiefer in Richtung der Längsachse ausgebildet ist, als der Grund des Gewindes,
c) einem Schraubenkopf, der an seiner Unterseite eine oder mehrere radiale Nuten besitzt, die in Verbindung mit der mindestens einen axialen Nut des Schraubenkörpers stehen,
d) einer Kappe mit einem Stift (als das Verbindungselement) an der Kappenunterseite, wobei der Stift einen für Flüssigkeiten durchlässigen Kanal besitzt, der mit einem Kanal in der Kappe flüssigkeitsdurchlässig verbunden oder verbindbar ist, der an einer Außenseite der Kappe in eine Einlassöffnung mündet, wobei die Kappe mit dem Stift in den Kanal der Knochenschraube reversibel eingesteckt oder einsteckbar ist, und
e) einem ersten Schlauch zur Fluidzuführung der flüssigkeitsdurchlässig mit der Einlassöffnung der Kappe verbunden ist,
f) wobei der erste Schlauch mit einer Auspressvorrichtung flüssigkeitsdurchlässig verbunden ist, wobei die Auspressvorrichtung zusammengesetzt ist aus:
   f1) einem Hohlzylinder in dem sich ein pharmazeutisches Fluid befindet,
   f2) einem im Hohlzylinder axial verschiebbaren Kolben, der ein Ende des Hohlzylinders verschließt,
   f3) mindestens einer flüssigkeitsdurchlässigen Austragsöffnung im geschlossenen Kopf des Hohlzylinders,
   f4) einem Ventilelement,
   f5) einem Federelement, das mit dem axial verschiebbaren Kolben verbunden ist, wobei mit dem gespannten Federelement der Kolben in Richtung der Austragsöffnung bewegbar ist, so dass das pharmazeutische Fluid im Hohlzylinder durch die Austragsöffnung und das Ventilelement in das Schlauchende des ersten Schlauchs einpressbar ist.

Durch diese beispielhafte erfindungsgemäße Vorrichtung ist es möglich, pharmazeutische Fluide über einen Zeitraum von Stunden bis zu mehreren Tagen kontinuierlich zu applizieren, ohne dass elektrisch betriebene Pumpsysteme notwendig sind. Die Vorrichtung kann so aus Kunststoffen gestaltet werden, dass diese vom mobilisierten Patienten mitgeführt werden kann.

Im Folgenden werden weitere Ausführungsbeispiele der Erfindung anhand von zwölf schematisch dargestellten Figuren erläutert, ohne jedoch dabei die Erfindung zu beschränken. Dabei zeigt:
Figur 1: eine schematische perspektivische Ansicht auf eine proximale Seite einer beispielhaften ersten erfindungsgemäßen Vorrichtung zur lokalen Applikation und zum Spülen von pharmazeutischen Fluiden;
Figur 2: eine schematische perspektivische Ansicht auf eine distale Seite der Vorrichtung nach Figur 1;
Figur 3: eine schematische Seitenansicht der Vorrichtung nach Figur 1 und 2;
Figur 4: eine schematische perspektivische Teilschnittansicht auf eine proximale Seite von Teilen einer beispielhaften zweiten erfindungsgemäßen Vorrichtung zur lokalen Applikation und zum Spülen von pharmazeutischen Fluiden;
Figur 5: eine schematische seitliche Teilschnittansicht der Teile nach Figur 4;
Figur 6: eine schematische seitliche Querschnittdarstellung der ersten erfindungsgemäßen Vorrichtung nach den Figuren 1 bis 3;
Figur 7: eine schematische perspektivische Ansicht auf eine proximale Seite aller Einzelteile der beispielhaften ersten erfindungsgemäßen Vorrichtung getrennt voneinander;
Figur 8: eine schematische Detailansicht als Ausschnittvergrößerung der Figur 6;
Figur 9: eine schematische Seitenansicht auf eine proximal anzuordnende Fördereinrichtung enthaltend einen Behälter zur Verbindung mit einer erfindungsgemäßen Vorrichtung;
Figur 10: eine schematische perspektivische Teilschnittansicht der Fördereinrichtung der Vorrichtung nach Figur 9;
Figur 11: eine schematische Teilschnittaufsicht der Fördereinrichtung nach Figur 10 im gespannten Zustand; und
Figur 12: eine schematische Teilschnittaufsicht der Fördereinrichtung nach Figur 10 im entspannten Zustand.

In den Figuren und in der folgenden Beschreibung zu den anhand der Figuren erläuterten Ausführungsbeispiele der vorliegenden Erfindung werden teilweise für unterschiedliche Ausführungsbeispiele und für unterschiedliche Einzelteile die gleichen Bezugszeichen bei gleichen oder gleichartigen Teilen verwendet, um die Vergleichbarkeit der Ausführungsbeispiele und die Lesbarkeit zu vereinfachen.

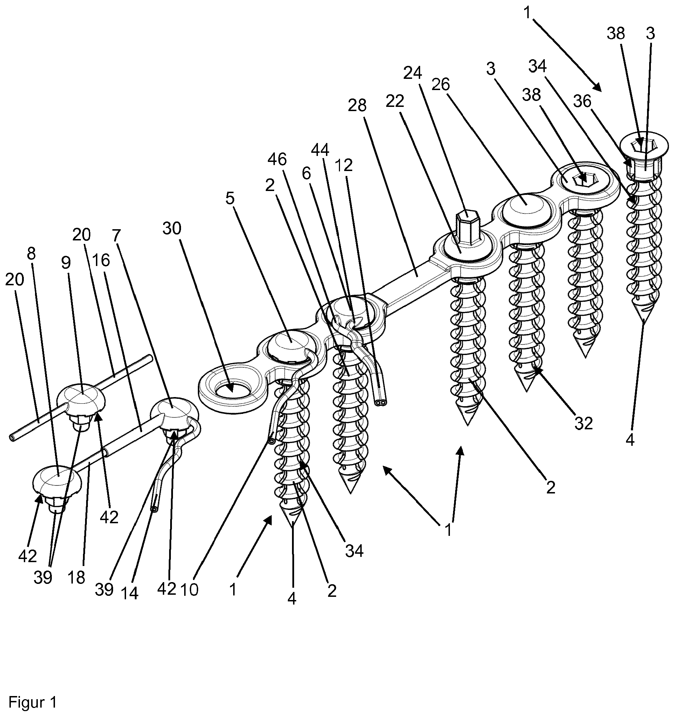

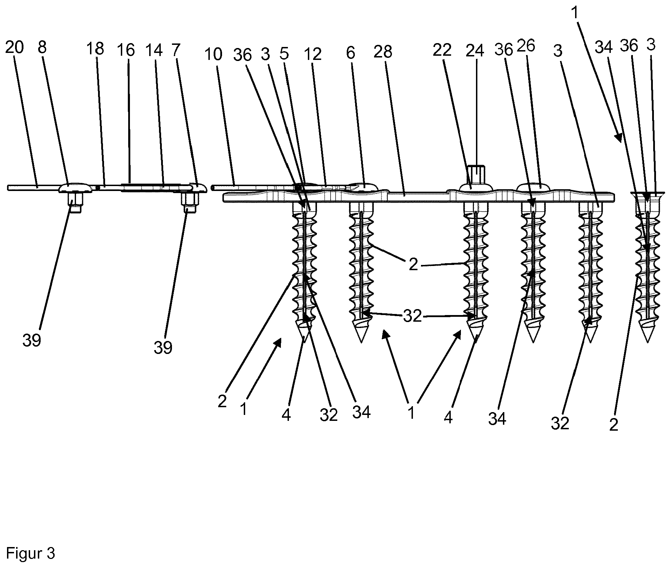

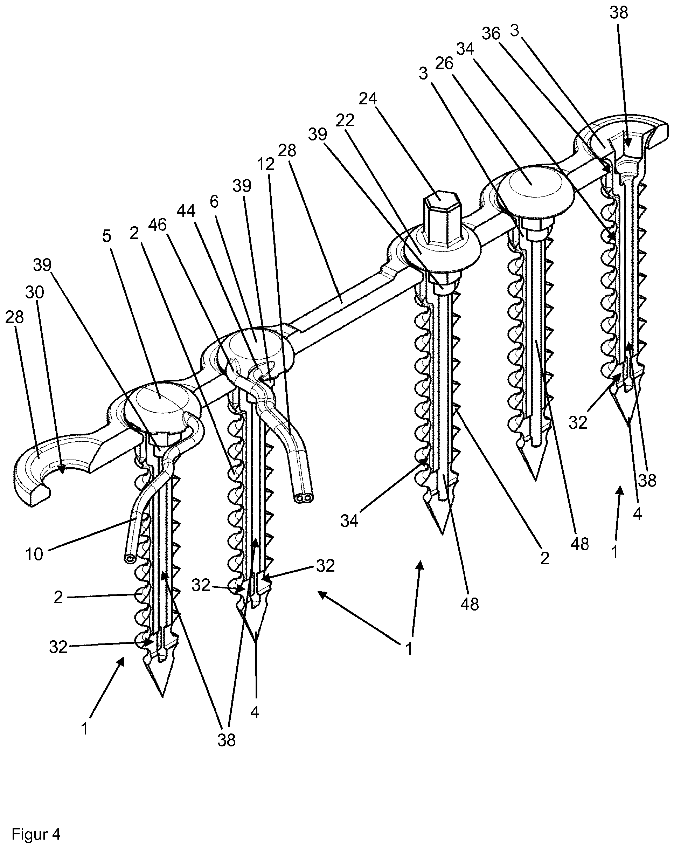

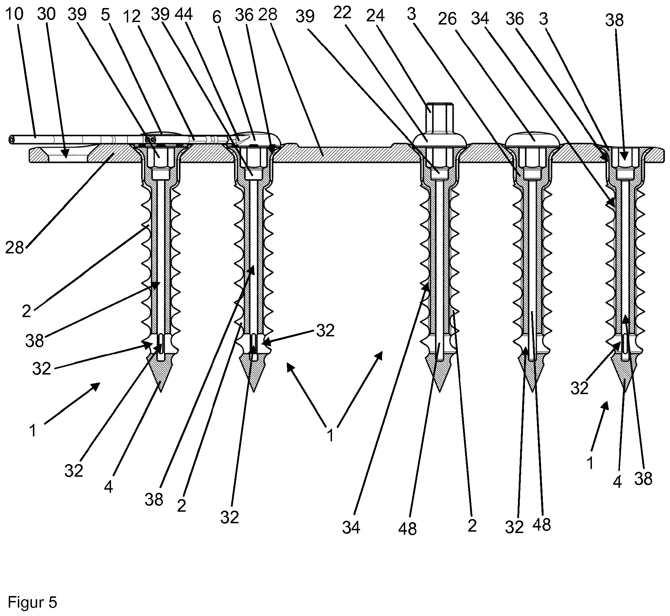

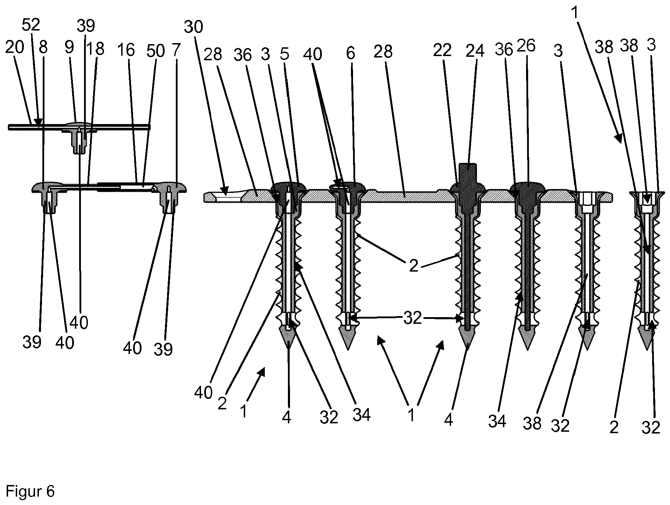

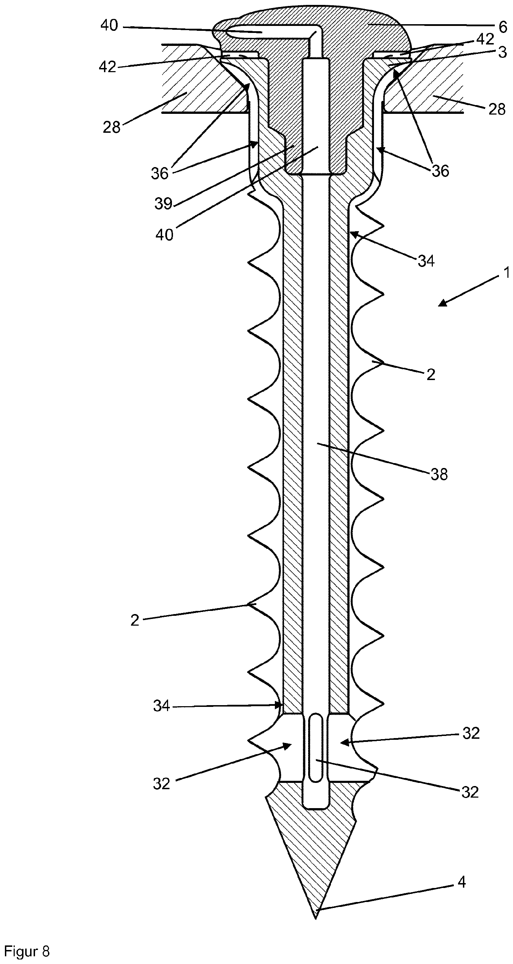

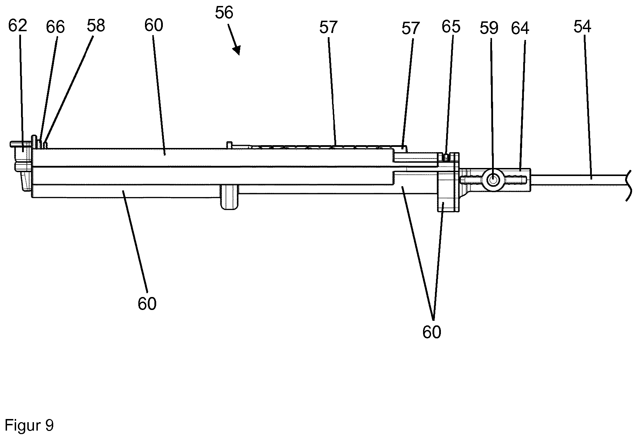

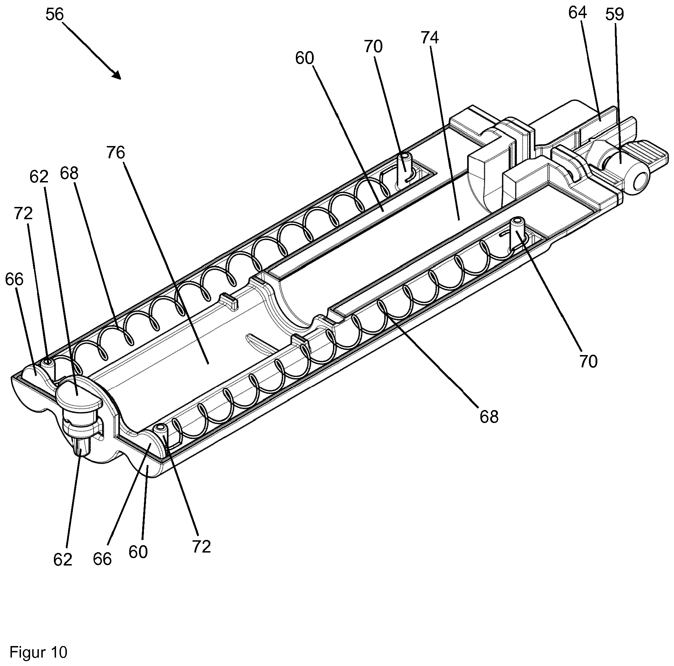

Die Figuren 1 bis 3 und 6 bis 8 zeigen eine erste beispielhafte erfindungsgemäße Vorrichtung und deren Teile in unterschiedlichen Darstellungen. Die Figuren 4 und 5 zeigen eine zweite beispielhafte erfindungsgemäße Vorrichtung in unterschiedlichen Darstellungen. Die Figuren 9 bis 12 zeigen schematische Darstellungen eine Fördereinrichtung, die ein Teil einer erfindungsgemäßen Vorrichtung sein kann.

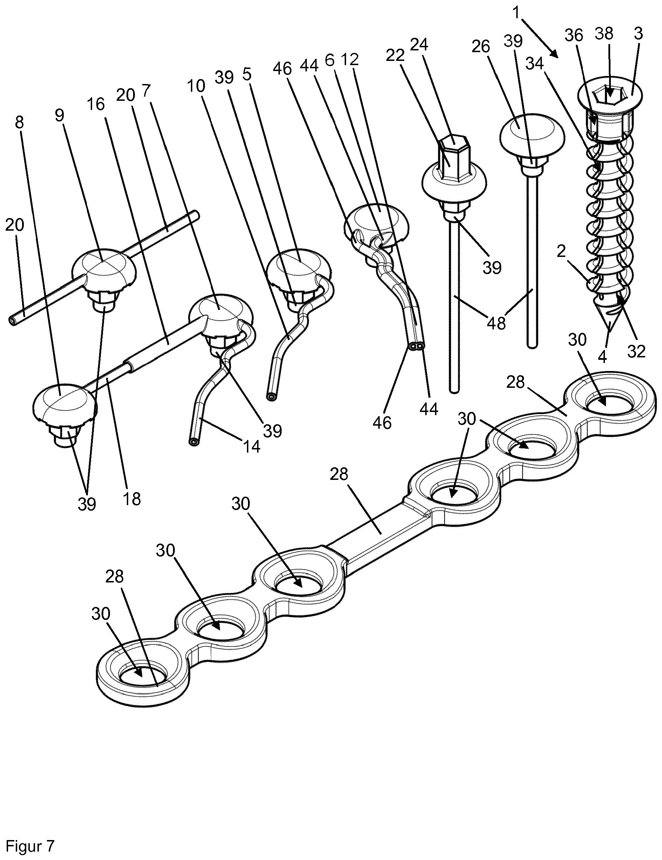

Die erste beispielhafte erfindungsgemäße Vorrichtung, die in den Figuren 1 bis 3 und 6 bis 8 gezeigt ist sowie die zweite beispielhafte erfindungsgemäße Vorrichtung, die in den Figuren 4 und 5 gezeigt ist, kann eine Mehrzahl von Knochenschrauben 1 umfassen. In den Figuren 1, 2, 3 und 6 sind jeweils sechs Knochenschrauben 1 gezeigt, in den Figuren 4 und 5 jeweils fünf, und Figur 7 zeigt eine der Knochenschrauben 1. Die Knochenschrauben 1 können jeweils baugleich sein und können aus einem Metall, wie beispielsweise Titan oder aus einem anderen biokompatiblen Material bestehen, aus denen ein Schraubenkörper der Knochenschrauben 1 besteht. Der Schraubenkörper umfasst vorliegend die gesamten Knochenschrauben 1.

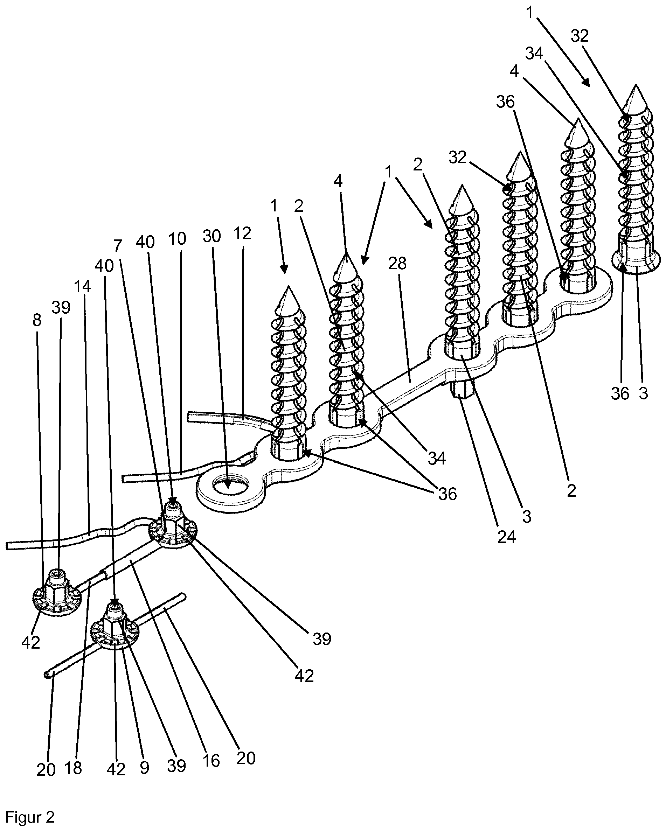

Die Knochenschrauben 1 weisen ein Außengewinde 2 auf, mit dem die Knochenschrauben 1 in einen Knochen (nicht gezeigt) eingeschraubt werden können. Hierzu kann das Außengewinde 2 selbstschneidend sein. An einem proximalen Ende der Knochenschrauben 1 (in den Figuren 1, 3 bis 8 oben und in Figur 2 unten) enden die Knochenschrauben 1 in einem Schraubenkopf 3. An einem dem Schraubenkopf 3 gegenüberliegenden distalen Schraubenende 4 der Knochenschraube 1 (in den Figuren 1, 3 bis 8 unten und in Figur 2 oben) können die Knochenschrauben 1 konisch spitz zulaufend ausgeformt sein.

Die erfindungsgemäßen Vorrichtungen können mehrere unterschiedliche Kappen 5, 6, 7, 8, 9 aufweisen, mit denen die Knochenschrauben 1 am Schraubenkopf 3 verschlossen oder abgedeckt werden können. Hierzu kann vorgesehen sein, dass die unterschiedlichen Kappen 5, 6, 7, 8, 9 an einer proximalen Seite des Schraubenkopfs 3 befestigt werden. Die Kappen 5, 6 sind in den Figuren 1 bis 8 in einem an die Schraubenköpfe 3 zweier Knochenschrauben 1 lösbar verbundenen Zustand gezeigt, während die Kappen 7, 8, 9 nicht mit Schraubenköpfen 3 von Knochenschrauben 1 verbunden dargestellt sind. Die gezeigten Kappen 5, 6, 7, 8, 9 stellen unterschiedliche mögliche Ausführungen dar. Es versteht sich, dass eine erfindungsgemäße Vorrichtung nur eine einzelne der Kappen 5, 6, 7, 8, 9 oder mehrere von nur einer Sorte der unterschiedlichen Kappen 5, 6, 7, 8, 9 oder eine beliebige Kombination der dargestellten Kappen 5, 6, 7, 8, 9 oder davon abgewandelter Kappen aufweisen kann. Es kann zur Realisierung der vorliegenden Erfindung also eine beliebige Auswahl von zur Umsetzung der vorliegenden Erfindung geeigneten Kappen 5, 6, 7, 8, 9 verwendet werden. Jede der Kappen 5, 6, 7, 8, 9 weist eine seitliche Einlassöffnung auf, durch die das pharmazeutische Fluid in die Kappe 5, 6, 7, 8, 9 eingeleitet werden kann.

Die in den Figuren 1 bis 8 dargestellten Kappen 5, 6, 7, 8, 9 unterscheiden sich voneinander durch unterschiedliche Möglichkeiten zum Durchleiten von pharmazeutischen Fluiden. An der Kappe 5 kann an der Einlassöffnung der Kappe 5 seitlich ein Schlauch 10 zur Fluidzuführung angeschlossen sein. An der Kappe 6 kann seitlich ein Doppelschlauch 12 angeschlossen sein, der einen Schlauch 44 zur Fluidableitung und einen Schlauch 46 zur Fluidzuführung umfasst, wobei der Schlauch 46 zur Fluidzuführung an die Einlassöffnung der Kappe 6 angeschlossen ist. Die Kappe 7 kann seitlich mit einem Schlauch 14 zur Fluidzuführung verbunden sein, der an der Einlassöffnung der Kappe 7 angeschlossen ist. Zusätzlich kann die Kappe 7 mit der Kappe 8 über zwei teleskopartig ineinandergesteckte Rohre 16, 18 verbunden sein. An der Kappe 7 kann dazu seitlich ein Rohr 16 mit einem größeren Durchmesser befestigt sein, während an der Kappe 8 seitlich ein Rohr 18 mit einem kleineren Durchmesser befestigt sein kann, wobei das Rohr 18 an die Einlassöffnung der Kappe 8 angeschlossen ist. Der Außendurchmesser des Rohrs 18 kann dem Innendurchmesser des Rohrs 16 entsprechen, so dass der Abstand zwischen der Kappe 7 und der Kappe 8 einstellbar ist und die beiden teleskopartig verbundenen Rohre 16, 18 eine Verbindung zum Durchleiten des pharmazeutischen Fluids zwischen den beiden Kappen 7, 8 bereitstellen. Die Kappe 9 kann an zwei gegenüberliegenden Seiten jeweils über ein Rohr 20 (eins zur Fluidzuführung, eins zur Fluidableitung) mit einem Reservoir des pharmazeutischen Fluids (nicht gezeigt, siehe aber beispielsweise Figur 9) verbunden werden oder mit anderen gleichartigen Kappen 9 oder mit anderen Kappen 5, 6, 7, 8 verbunden werden. Hierzu ist eines der Rohre 20 an die Einlassöffnung der Kappe 9 angeschlossen und das jeweils andere Rohr 20 zur Fluidableitung vorgesehen. Auf diese Weise lassen sich eine Mehrzahl gleicher oder unterschiedlicher Kappen 5, 6, 7, 8, 9 miteinander an ein Reservoir für das pharmazeutische Fluid (siehe beispielsweise Figur 9) seriell oder auch parallel anschließen. Das pharmazeutische Fluid kann dann bevorzugt durch die Kappen 5, 6, 7, 8, 9 und durch die angeschlossenen Knochenschrauben 1 fließen und besonders bevorzugt auch wieder aus diesen abgeleitet werden.

Um die Knochenschrauben 1 verschlossen zu halten und gegebenenfalls auch leichter in einen Knochen einschrauben zu können, kann eine Verschlusskappe 22 mit einem proximal außen liegenden Sechskant 24 oder eine Verschlusskappe 26 mit ebener Abdeckung in die Knochenschrauben 1 eingesteckt sein. Die Verschlusskappen 22, 26 dienen vor allem dem Versiegeln der Knochenschrauben 1, wenn keine der Kappen 5, 6, 7, 8, 9 an der Knochenschraube 1 befestigt ist.

Die Vorrichtung kann eine Osteosyntheseplatte 28 aufweisen. Die Osteosyntheseplatte 28 muss allerdings nicht Teil der Vorrichtung sein. Es können stattdessen auch herkömmliche Osteosyntheseplatten mit einer erfindungsgemäßen Vorrichtung aufweisend Knochenschrauben 1 und Kappen 5, 6, 7, 8, 9 verwendet werden. Die Osteosyntheseplatte 28 kann sechs Löcher 30 zum Verschrauben der Osteosyntheseplatte 28 mit den Knochenschrauben 1 aufweisen. Eine andere Anzahl von Löchern 30 ist selbstverständlich ebenfalls möglich, wobei zum Fixieren einer Fraktur zumindest zwei Löcher 30 bevorzugt zumindest vier Löcher 30 vorhanden sein sollten. Die Löcher 30 können einen Innendurchmesser aufweisen, der kleiner ist als der Außendurchmesser der Schraubenköpfe 3, der aber größer ist als der Außendurchmesser des Außengewindes 2, so dass die Knochenschrauben 1 bis auf die Schraubenköpfe 3 durch die Löcher 30 der Osteosyntheseplatte 28 gesteckt werden können und die Osteosyntheseplatte 28 mit den Knochenschrauben 1 fixierbar ist. Die Osteosyntheseplatte 28 kann im Bereich der Löcher 30 eine Geometrie aufweisen, die zur distalen Oberfläche der Schraubenköpfe 3 passend geformt ist. Die Knochenschrauben 1 können aber auch ohne weiteres ohne eine Osteosyntheseplatte 28 zum Verbinden und Fixieren mehrerer Teile eines frakturierten Knochens verwendet werden. Daher muss eine erfindungsgemäße Vorrichtung auch keine Osteosyntheseplatte 28 aufweisen.

Die Knochenschrauben 1 können im Bereich des distalen Schraubenendes 4 vier sich in radialer Richtung der Knochenschraube 1 erstreckende Fluidaustrittsöffnungen 32 aufweisen. Die Fluidaustrittsöffnungen 32 können in vier axiale Nuten 34 in dem Außengewinde 2 der Knochenschrauben 2 münden, die sich bis zum Schraubenkopf 3 erstrecken und dort in zumindest vier radiale Nuten 36 am Schraubenkopf 3 münden. Der Nutgrund der axialen Nuten 34 kann dabei tiefer sein als der Gewindegrund des Außengewindes 2, damit das pharmazeutische Fluid entlang der axialen Nuten 34 fließen kann. Die radialen Nuten 36 können sich bis zum radialen Rand des Schraubenkopfs 3 erstrecken. Die radialen Nuten 36 und die Osteosyntheseplatte 28 bilden so einen Kanal zum Ableiten beziehungsweise zum Weiterleiten des pharmazeutischen Fluids, wenn die Knochenschrauben 1 mit der Osteosyntheseplatte 28 verbunden sind. Der Schlauch 44 zur Fluidableitung oder das Rohr 16 oder eines der Rohre 20 kann hierzu mit den axialen Nuten 34 verbunden sein. Analog können die axialen Nuten 34 mit dem umgebenden Knochengewebe jeweils einen Kanal zum Durchleiten des pharmazeutischen Fluids bilden. Anstelle von vier Fluidaustrittsöffnungen 32 und vier axialen Nuten 34 kann auch nur eine Fluidaustrittsöffnung und eine axiale Nut oder eine beliebige andere Anzahl von Fluidaustrittsöffnungen und axialen Nuten verwendet werden. Mehrere Fluidaustrittsöffnungen 32 werden jedoch bevorzugt, damit das pharmazeutische Fluid in unterschiedliche radiale Richtungen im Bohrloch im Knochen austreten kann und allseitig zur Behandlung zur Verfügung steht.

Im Inneren der Knochenschrauben 1 kann sich ein durchgehender Kanal 38 in axialer Richtung vom Schraubenkopf 3 bis zu den Fluidaustrittsöffnungen 32 erstrecken. Der Kanal 38 in der Knochenschraube 1 dient dazu, das pharmazeutische Fluid von dem Schraubenkopf 3 her durch die Knochenschraube 1 hindurch zu den Fluidaustrittsöffnungen 32 zu leiten. Der axiale Kanal 38 kann im Schraubenkopf 3 einen vergrößerten Durchmesser aufweisen, um eine Verbindung zu den Kappen 5, 6, 7, 8, 9 zu ermöglichen. Der axiale Kanal 38 kann im Schraubenkopf 3 ein Sechskantloch oder eine andere gebrochene Symmetrie als Antrieb der Knochenschraube 1 aufweisen, um die Knochenschrauben 1 bedienen zu können und/oder um die Kappen 5, 6, 7, 8, 9 und die Verschlusskappen 22, 26 an dem Schraubenkopf 3 lösbar befestigen zu können. Hierzu können die Kappen 5, 6, 7, 8, 9 und die Verschlusskappen 22, 26 an ihrer proximalen Unterseite Verbindungselemente 39 in Form vom Vorsprüngen aufweisen. Die Verbindungselemente 39 können stiftförmige Vorsprünge bilden. Die Verbindungselemente 39 können eine Negativform des Kanals 38 im Schraubenkopf 3 bilden, beispielsweise eine Sechskantgeometrie. Hierdurch können die Kappen 5, 6, 7, 8, 9 und die Verschlusskappen 22, 26 durch Einstecken in die Schraubenköpfe 3 der Knochenschrauben 1 an den Schraubenköpfen 3 befestigt werden.

Im Inneren der Kappen 5, 6, 7, 8, 9 können Kanäle 40 angeordnet sein, die sich von den Einlassöffnungen der Kappen 5, 6, 7, 8, 9 bis in die Verbindungselemente 39 der Kappen 5, 6, 7, 8, 9 erstrecken. Die Kanäle 40 der Kappen 5, 6, 7, 8, 9 sind nur in den Querschnittzeichnungen der Figuren 2, 6 und 8 zu erkennen, aber auch innerhalb der Kappen 5, 6, 7, 8, 9 nach den Figuren 1 und 3 bis 5 und 7 angeordnet, dort allerdings in den Abbildungen nicht erkennbar. Die Kanäle 40 in den Kappen 5, 6, 7, 8, 9 münden dabei vorzugsweise derart in Öffnungen in den Verbindungselementen 39, dass eine flüssigkeitsdurchlässige Verbindung zwischen der Einlassöffnung der Kappen 5, 6, 7, 8, 9 und den Fluidaustrittsöffnungen 32 der Knochenschrauben 1 vorhanden ist, wenn die Kappen 5, 6, 7, 8, 9 mit den Verbindungselementen 39 in den breiten Teilen der Kanäle 38 der Knochenschrauben 1 eingesteckt und dort lösbar befestigt sind. Hierdurch ist es möglich, das pharmazeutische Fluid durch die Kappen 5, 6, 7, 8, 9 in den Kanal 38 einzuleiten und von dort bis zu den Fluidaustrittsöffnungen 32 innerhalb des Schraubenkörpers beziehungsweise innerhalb der Knochenschrauben 1 zu leiten.

An der distalen Unterseite der Kappen 5, 6, 7, 8, 9 können mehrere radial ausgerichtete Nuten 42 angeordnet sein. Diese radialen Nuten 42 der Kappen 5, 6, 7, 8, 9 können flüssigkeitsdurchlässig mit den radialen Nuten 36 an den Schraubenköpfen 3 verbunden sein, wenn die Kappen 5, 6, 7, 8, 9 mit den Schraubenköpfen 3 verbunden sind. Die Nuten 42 können ferner mit dem Schlauch 44 zur Fluidableitung oder mit dem Rohr 20 oder mit dem Rohr 16 flüssigkeitsdurchlässig verbunden sein. Hierdurch kann das pharmazeutische Fluid durch die radialen Nuten 42 abgeführt werden und gegebenenfalls zu einer weiteren Kappe 5, 6, 7, 8, 9 geleitet werden.

An der distalen Seite der Verbindungselemente 39 der Verschlusskappen 22, 26 können Stifte 48 angeordnet sein. Diese Stifte 48 haben vorzugsweise eine Negativform der Kanäle 38 und füllen so die Kanäle 38 der Knochenschrauben 1 vollständig aus, so dass keine Fremdkörper in die Kanäle 38 eindringen können, wenn die Stifte 48 die Kanäle 38 ausfüllen. Die Verschlusskappe 22 wird vorzugsweise dazu verwendet, den Kanal 38 einer Knochenschraube 1 zu verschließen und um die Knochenschraube 1 einzuschrauben, während die Verschlusskappe 26 vorzugsweise dazu verwendet wird, den Kanal 38 einer Knochenschraube 1 zu verschließen, die im eingeschraubten Zustand nicht mehr zum Spülen verwendet wird. Die Verschlusskappe 26 kann also zur Anwendung unter dem Weichgewebe vorgesehen sein, während die Verschlusskappe 22 beim Einschrauben einer Knochenschraube 1 verwendet werden kann.

Die Rohre 16, 18 können in ihrem Inneren eine Leitung 50 bilden, durch die das pharmazeutische Fluid von der Kappe 7 zur Kappe 8 geleitet werden kann. Ebenso können die Rohre 20 in ihrem Inneren Leitungen 52 bilden, durch die das pharmazeutische Fluid in die Kappe 9 eingeleitet oder von der Kappe 9 weitergeleitet werden kann.

An einem Schlauch 54 zur Fluidzuleitung des pharmazeutischen Fluids in eine Kappe 5, 6, 7, 8, 9 kann eine Fördereinrichtung 56 angeordnet sein oder angeschlossen werden, wie sie schematisch in den Figuren 9 bis 12 dargestellt ist. In die Fördereinrichtung 56 kann ein Behälter 57 in Form einer Spritze mit einem Kolben 58 zum Auspressen des Inhalts der Spritze eingesetzt werden beziehungsweise eingesetzt sein. Der Kolben 58 kann in axialer Richtung in der Spritze verschiebbar angeordnet sein und fluiddicht gegen die Innenwand des Behälters 57 abgedichtet sein. Die Fördereinrichtung 56 kann ein Gehäuse 60 aus einem Plastik aufweisen, das das Innere der Fördereinrichtung 56 vollständig oder teilweise nach außen verschließen kann. Ein Sicherungsbolzen 62 kann in eine Öffnung am proximalen Ende des Gehäuses 60 eingesteckt sein.

Auf der distalen Seite der Fördereinrichtung 56 kann eine Halterung 64 zur Befestigung des proximalen Endes des Schlauchs 54 angeordnet sein. Hierzu kann an dem Schlauch 54 eine Halterungsscheibe 65 befestigt sein, die in die Halterung 64 eingreifen kann.

In der Fördereinrichtung 56 kann eine Förderplatte 66 zum Einpressen des Kolbens 58 in den Behälter 57 angeordnet sein. Mit dem Sicherungsbolzen 62 kann die Förderplatte 66 gegen das Gehäuse 60 arretiert werden. Hierzu kann eine Öse an der proximalen Seite der Förderplatte 66 aus dem Gehäuse 60 der Fördereinrichtung 56 ragen und durch Einstecken des Sicherungsbolzens 62 die Förderplatte 66 gegen das Gehäuse 60 arretiert werden. Die Förderplatte 66 kann von zwei gespannten Federn 68 angetrieben werden. Die beiden Federn 68 stellen ein Energiespeicherelement dar, in denen zumindest die Energie gespeichert ist, die zum Auspressen eines pharmazeutischen Fluids aus dem Behälter 57 und durch den Schlauch 54 und durch die an den Schlauch 54 angeschlossenen Kanäle 38, 40 (gegebenenfalls auch durch angeschlossene Leitungen 50, 52), durch die angeschlossenen und im Knochen eines Patienten eingeschraubten Knochenschraube(n) 1 hindurch, aus diesen heraus und entlang der Nuten 34, 36 bis zum Schraubenkopf 3 oder am Schraubenkopf 3 vorbei notwendig ist. Bevorzugt kann die Federkraft der Federn 68 auch dazu ausreichen, das pharmazeutische Fluid durch einen angeschlossenen Schlauch 44 zur Fluidableitung oder entsprechende Rohre abzuführen und wieder aus dem Patienten abzuleiten.

Die Federn 68 können an ihren distalen Enden mit Stiften 70 am Gehäuse 60 befestigt sein. An ihren proximalen Enden können die Federn 68 mit Stiften 72 an der Förderplatte 66 befestigt sein. Die Federn 68 können so zwischen den Stiften 70 und den Stiften 72 gespannt sein.

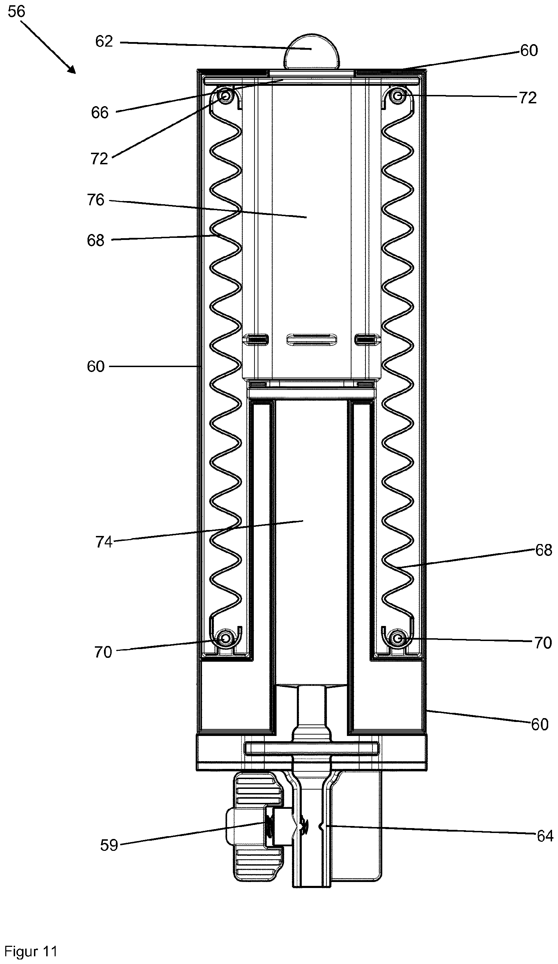

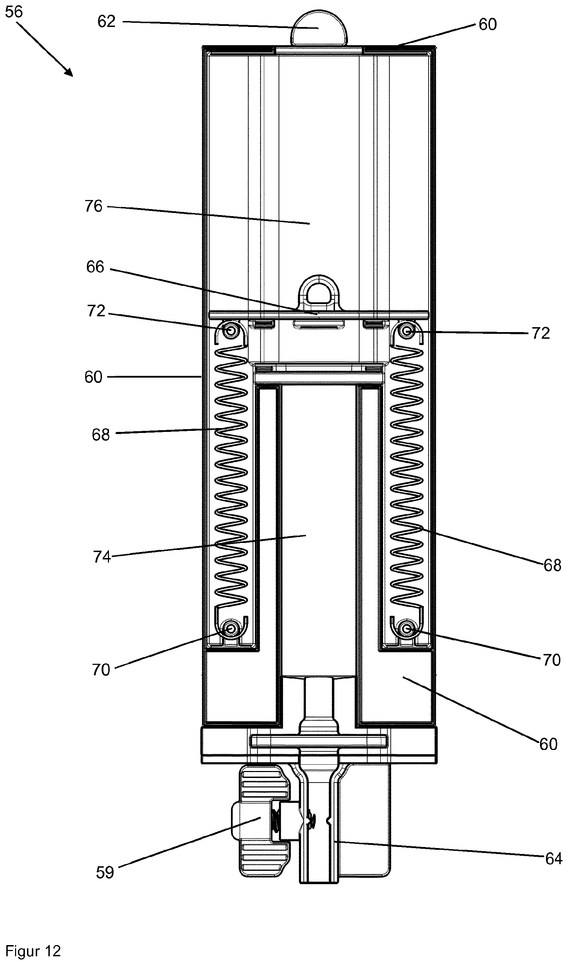

Im Inneren des Gehäuses 60 kann eine Aufnahme 74 für den Behälter 60 und ein Hubraum 76 für den Kolben 58 ausgeformt sein. Der Behälter 60 kann durch die Form der Aufnahme 74 in der Fördereinrichtung 56 fixiert werden. Die Förderplatte 66 kann auf diese Weise von den Federn 68 vom proximalen Ende bis zum distalen Ende des Hubraums 76 gezogen werden (siehe Figuren 11 und 12). Der Kolben 58 kann in der Fördereinrichtung 56 mit der Förderplatte 66 angetrieben durch die Federn 68 in den Behälter 60 gepresst werden, wenn der Sicherungsbolzen 62 entfernt wurde und das Ventilelement 59 geöffnet ist. Dadurch kann ein in dem Behälter 57 enthaltenes pharmazeutisches Fluid aus dem Behälter 57 und durch den Schlauch 54 und durch angeschlossene Kappen 5, 6, 7, 8, 9, 22, 26 und Knochenschrauben 1 gedrückt werden. Mit dem auf das pharmazeutische Fluid wirkenden Druck kann gegebenenfalls auch ein Spülkreislauf des pharmazeutischen Fluids angetrieben werden, der aus dem Knochen des Patienten oder aus dem Körper des Patienten heraus führt.

Als das zu applizierende pharmazeutische Fluid kann je nach Anwendung beispielsweise eine wässrige Lösung umfassend wenigstens ein Antibiotikum und/oder wenigstens ein Antimykotikum verwendet werden. Ferner kann das medizinische Fluid auch wenigstens ein Zytostatikum und/oder wenigstens ein Chemotherapeutikum enthalten.

Für eine medizinische Anwendung der erfindungsgemäßen Vorrichtungen können die Knochenschrauben und vorzugsweise auch die Kappen 5, 6, 7, 8, 9 gegebenenfalls vorhandene Osteosyntheseplatten 28 aus biokompatiblen Materialien aufgebaut werden, in denen Röntgenopaker enthalten sind, so dass deren Lage mit bildgebenden Röntgenverfahren bestimmbar ist.

Im Folgenden wird eine beispielhafte Anwendung der erfindungsgemäßen Vorrichtungen geschildert. Die Knochenschrauben 1 werden in einen Knochen (nicht gezeigt) eingeschraubt und dabei die Position der Knochenschrauben 1 relativ zueinander durch eine Osteosyntheseplatte 28 festgelegt. Dabei können zwei oder mehr Teile eines frakturierten Knochens zueinander fixiert werden. Die Knochenschrauben 1 können dabei mit Verschlusskappen 22, 26 verschlossen sein. Die Knochenschrauben 1 können dabei durch die Löcher 30 der Osteosyntheseplatte 28 geführt sein und bis zum Anschlag der Schraubenköpfe 3 an die Osteosyntheseplatte 28 eingeschraubt werden.

Anschließend werden die Verschlusskappen 22, 26 entfernt und dafür Kappen 5, 6, 7, 8, 9 in die freien Kanäle 38 der Knochenschrauben 1 eingesteckt und dort verbunden. Die Kappen 5, 6, 7, 8, 9 stellen eine flüssigkeitsdurchlässige Verbindung zwischen den Einlassöffnungen der Kappen 5, 6, 7, 8, 9 und den Kanälen 38 der Knochenschrauben 1 her. Über die Leitungen 50, 52 der Rohre 16, 18, 20 oder über die Schläuche 10, 12, 14, 44, 46, 54 können die Einlassöffnungen der Kappen 5, 6, 7, 8, 9 miteinander und mit dem Behälter 57 verbunden werden. Die Fördereinrichtung 56 ist hierbei außerhalb des Körpers des Patienten angeordnet und der Schlauch 54 zur Fluidzuleitung in den Patienten hineingeführt. Das pharmazeutische Fluid kann mit der Fördereinrichtung 56 über den Schlauch 54 und wenigstens einen der Schläuche 10, 14, 46 zur Fluidzuführung in wenigstens eine der Kappen 5, 6, 7, 8, 9 eingepresst werden. Von dort kann das pharmazeutische Fluid durch den Kanal 40 oder die Kanäle 40 zumindest einer der Kappen 5, 6, 7, 8, 9 in den Kanal 38 wenigstens einer Knochenschraube 1 und dort aus den Fluidaustrittsöffnungen 32 der wenigstens einen Knochenschraube 1 herausgedrückt werden. Dort fließt das pharmazeutische Fluid entlang der axialen Nuten 34 am Kochen entlang zu den radialen Nuten 36, dort durch die radialen Nuten 36 in die radialen Nuten 42 der Kappen 5, 6, 7, 8, 9. Von dort kann das pharmazeutische Fluid durch einen Schlauch 44 zur Fluidableitung oder durch ein Rohr 16, 20 ableitet werden. Das pharmazeutische Fluid kann dabei aus dem Körper herausgeleitet werden oder durch eine weitere Kappe 5, 6, 7, 8, 9 und eine weitere Knochenschraube 1 geleitet werden. Das abgeleitete pharmazeutische Fluid kann zur Prüfung des Behandlungserfolgs untersuchte werden und abhängig von dem Ergebnis die Behandlung durch eine Veränderung der Zusammensetzung und/oder der Durchflussmenge des pharmazeutischen Fluids angepasst werden. Alternativ tritt das pharmazeutische Fluid aus den radialen Nuten 42 der Kappen 5, 6, 7, 8, 9 aus in wird im Körper abseits von dem zu behandelnden Knochen resorbiert.

Durch die erfindungsgemäße Vorrichtung kann also eine Spülung mit einem pharmazeutischen Fluid erzeugt werden, wobei das pharmazeutische Fluid jederzeit an die Behandlungssituation angepasst werden kann. Durch die Nuten 34, 36, 42 kann sich kein Druck des pharmazeutischen Fluids innerhalb des Knochens aufbauen. Hierdurch können eine Reizung des zu behandelnden Gewebes und Embolien verhindert werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Knochenschraube
- 2: Außengewinde
- 3: Schraubenkopf
- 4: Distales Schraubenende
- 5, 6: Kappe
- 6, 7, 9: Kappe
- 10: Schlauch zur Fluidzuführung
- 12: Doppelschlauch zur Fluidzuführung und zur Fluidableitung
- 14: Schlauch zur Fluidzuführung
- 16, 18: Rohr
- 20: Rohr
- 22: Verschlusskappe
- 24: Sechskant
- 26: Verschlusskappe
- 28: Osteosyntheseplatte
- 30: Loch
- 32: Fluidaustrittsöffnung
- 34, 36: Nut
- 38: Kanal
- 39: Verbindungselement
- 40: Kanal
- 42: Nut
- 44: Schlauch zur Fluidableitung
- 46: Schlauch zur Fluidzuführung
- 48: Stift
- 50, 52: Leitung
- 54: Schlauch zur Fluidzuführung
- 56: Fördereinrichtung
- 57: Behälter
- 58: Kolben
- 59: Ventilelement
- 60: Gehäuse
- 62: Verschlussbolzen
- 64: Halterung
- 65: Halterungsscheibe
- 66: Förderplatte
- 68: Feder
- 70: Stift
- 72: Stift
- 74: Aufnahme für Behälter
- 76: Hubraum für Kolben

## Patentansprüche

1. Vorrichtung zur lokalen Applikation von und/oder zum Spülen mit pharmazeutischen Fluiden, die Vorrichtung aufweisend
mindestens eine Knochenschraube (1), die mindestens eine Knochenschraube (1) aufweisend einen Schraubenkörper, ein Außengewinde (2) und einen proximalen Schraubenkopf (3), wobei mindestens ein Kanal (38) im Schraubenkörper angeordnet ist, wobei der mindestens eine Kanal (38) flüssigkeitsdurchlässig ist, vom Schraubenkopf (3) ausgeht und in wenigstens eine Fluidaustrittsöffnung (32) im Schraubenkörper mündet, wobei die wenigstens eine Fluidaustrittsöffnung (32) vom Schraubenkopf (3) in distaler Richtung beabstandet ist,
wenigstens eine das Außengewinde (2) der Knochenschraube (1) durchbrechende axiale Nut (34), die sich von der wenigstens einen Fluidaustrittsöffnung (32) im Schraubenkörper bis zum Schraubenkopf (3) erstreckt, wobei ein Nutgrund der wenigstens einen axialen Nut (34) im Schraubenkörper tiefer ist als ein Grund des Außengewindes (2),
mindestens eine Kappe (5, 6, 7, 8, 9) mit einem Verbindungselement (39) zum lösbaren Verbinden der mindestens einen Kappe (5, 6, 7, 8, 9) mit dem proximalen Schraubenkopf (3) der mindestens einen Knochenschraube (1), wobei in der mindestens einen Kappe (5, 6, 7, 8, 9) ein flüssigkeitsdurchlässiger Kanal (40) angeordnet ist, wobei der Kanal (40) in der mindestens einen Kappe (5, 6, 7, 8, 9) in den mindestens einen Kanal (38) der mindestens einen Knochenschraube (1) mündet, wenn die mindestens eine Kappe (5, 6, 7, 8, 9) über das Verbindungselement (39) mit der mindestens einen Knochenschraube (1) lösbar verbunden ist, und wobei der Kanal (40) an einer Einlassöffnung in der mindestens einen Kappe (5, 6, 7, 8, 9) beginnt, **dadurch gekennzeichnet, dass**
die Vorrichtung mindestens einen Schlauch (10, 14, 46, 54) zur Fluidzuführung aufweist, der flüssigkeitsdurchlässig mit der Einlassöffnung an einer der mindestens einen Kappe (5, 6, 7, 8, 9) verbunden oder verbindbar ist, so dass ein pharmazeutisches Fluid mit einem Druck durch den mindestens einen Schlauch (10, 14, 46, 54) zur Fluidzuführung, durch den Kanal (40) der mindestens einen Kappe (5, 6, 7, 8, 9) und durch den mindestens einen Kanal (38) der mindestens einen Knochenschraube (1) aus der wenigstens einen Fluidaustrittsöffnung (32) drückbar ist, wenn der mindestens eine Schlauch (10, 14, 46, 54) zur Fluidzuführung mit der mindestens einen Kappe (5, 6, 7, 8, 9) und die mindestens eine Kappe (5, 6, 7, 8, 9) über das Verbindungselement (39) mit der mindestens einen Knochenschraube (1) verbunden sind.

2. Vorrichtung nach Anspruch 1, **dadurch gekennzeichnet, dass**
die mindestens eine Knochenschraube (1) wenigstens eine radiale Nut (36) aufweist, die in der Oberfläche des Schraubenkopfs (3) der mindestens einen Knochenschraube (1) angeordnet ist und die mit der wenigstens einen axialen Nut (34) im Schraubenkörper verbunden ist.

3. Vorrichtung nach Anspruch 1 oder 2, **dadurch gekennzeichnet, dass** sich die wenigstens eine das Außengewinde (2) der Knochenschraube (1) durchbrechende axiale Nut (34) von der wenigstens einen Fluidaustrittsöffnung (32) am distalen Schraubenkörper bis zu einer distalen Seite des Schraubenkopfs (3) erstreckt, wobei bevorzugt sich die wenigstens eine axiale Nut (34) bis auf die radial außen liegende Seite des Schraubenkopfs (3) erstreckt.

4. Vorrichtung nach einem der vorangehenden Ansprüche, **dadurch gekennzeichnet, dass**
das Verbindungselement (39) ein Vorsprung an einer zum Schraubenkopf (3) weisenden Kappenunterseite ist, wobei der Vorsprung den für Flüssigkeiten durchlässigen Kanal (40) in der mindestens einen Kappe (5, 6, 7, 8, 9) umfasst, wobei die mindestens eine Kappe (5, 6, 7, 8, 9) mit dem Vorsprung in den mindestens einen Kanal (38) der mindestens einen Knochenschraube (1) reversibel eingesteckt ist oder einsteckbar ist, wobei bevorzugt der Vorsprung im Zentrum der Kappenunterseite angeordnet ist, besonders bevorzugt axial entlang einer Symmetrieachse der mindestens einen Kappe (5, 6, 7, 8, 9) angeordnet ist.

5. Vorrichtung nach Anspruch 4, **dadurch gekennzeichnet, dass** die mindestens eine Kappe (5, 6, 7, 8, 9) an der zum Schraubenkopf (3) weisenden Kappenunterseite zumindest eine sich in radialer Richtung von dem Vorsprung nach außen erstreckende Nut (42) aufweist, wobei die zumindest eine Nut (42) der mindestens einen Kappe (5, 6, 7, 8, 9) sich bevorzugt bis zum radialen Rand der mindestens einen Kappe (5, 6, 7, 8, 9) erstreckt und/oder ein Schlauch (44) zur Fluidableitung mit der zumindest einen Nut (42) der mindestens einen Kappe (5, 6, 7, 8, 9) flüssigkeitsdurchlässig verbunden ist.

6. Vorrichtung nach einem der vorangehenden Ansprüche, **dadurch gekennzeichnet, dass**
der mindestens eine Schlauch (10, 14, 46, 54) zur Fluidzuführung mit einem Fluidreservoir flüssigkeitsdurchlässig verbunden oder verbindbar ist, wobei ein pharmazeutisches Fluid aus dem Fluidreservoir unter Druck in den mindestens einen Schlauch (10, 14, 46, 54) zur Fluidzuführung durch den Kanal (40) der mindestens einen Kappe (5, 6, 7, 8, 9) und den mindestens einen Kanal (38) der mindestens einen Knochenschraube (1) pressbar ist.

7. Vorrichtung nach einem der vorangehenden Ansprüche, **dadurch gekennzeichnet, dass**
der mindestens eine Kanal (38) im Schraubenkörper außer an der wenigstens einen Fluidaustrittsöffnung (32) nicht mit der wenigstens einen axialen Nut (34) im Schraubenkörper verbunden ist.

8. Vorrichtung nach einem der vorangehenden Ansprüche, **dadurch gekennzeichnet, dass**
die mindestens eine Kappe (5, 6, 7, 8, 9) kuppelförmig mit einer bis auf das Verbindungselement (39) planen Unterseite ausgebildet ist und/oder die mindestens eine Kappe (5, 6, 7, 8, 9) den Schraubenkopf (3) vollständig abdeckt, bevorzugt die mindestens eine Kappe (5, 6, 7, 8, 9) den Schraubenkopf (3) überlappt.

9. Vorrichtung nach einem der vorangehenden Ansprüche, **dadurch gekennzeichnet, dass**
die mindestens eine Kappe (5, 6, 7, 8, 9) eine Öffnung oder mehrere Öffnungen zum Ableiten von Fluiden aufweist, die mit einem Schlauch (44) zur Fluidableitung verbindbar oder verbunden sind, wobei die Öffnung oder die Öffnungen vorzugsweise an einer zum Schraubenkopf (3) weisenden Kappenunterseite der mindestens einen Kappe (5, 6, 7, 8, 9) angeordnet ist oder sind und/oder
der mindestens eine Schlauch (46) zur Fluidzuführung und der Schlauch (44) zur Fluidableitung in Längsrichtung miteinander verbunden sind, bevorzugt parallel nebeneinander miteinander verbunden sind oder koaxial zueinander angeordnet sind.

10. Vorrichtung nach einem der vorangehenden Ansprüche, **dadurch gekennzeichnet, dass**
die Vorrichtung zumindest eine Osteosyntheseplatte (28) aufweist und/oder die mindestens eine Kappe (5, 6, 7, 8, 9) wenigstens ein Rastelement aufweist, mit der die mindestens eine Kappe (5, 6, 7, 8, 9) mit einer Osteosyntheseplatte (28) zu verrasten ist, wobei bevorzugt das wenigstens eine Rastelement an einer zum Schraubenkopf (3) weisenden Kappenunterseite der mindestens einen Kappe (5, 6, 7, 8, 9) angeordnet ist.

11. Vorrichtung nach einem der vorangehenden Ansprüche, **dadurch gekennzeichnet, dass**
der mindestens eine Schlauch (10, 14, 46, 54) zur Fluidzuführung eine maximale radiale Dehnung von 5 Prozent, insbesondere eine maximale radiale Dehnung von 1 Prozent, bei einem Innendruck von 5 bar aufweist, wobei bevorzugt auch ein Schlauch (44) zur Fluidableitung eine maximale radiale Dehnung von 5 Prozent, insbesondere eine maximale radiale Dehnung von 1 Prozent, bei einem Innendruck von 5 bar aufweist.

12. Vorrichtung nach einem der vorangehenden Ansprüche, **dadurch gekennzeichnet, dass**
die mindestens eine Kappe (5, 6, 7, 8, 9) mindestens zwei Kappen (5, 6, 7, 8, 9) sind und die mindestens eine Knochenschraube (1) mindestens zwei Knochenschrauben (1) sind, wobei die mindestens zwei Kappen (5, 6, 7, 8, 9) mit ihren Verbindungselementen (39) in zwei unterschiedliche der mindestens zwei Knochenschrauben (1) befestigt sind, wobei die mindestens zwei Kappen (5, 6, 7, 8, 9) durch ein Rohr (16, 18, 20) oder zwei Rohre (16, 18, 20) flüssigkeitsdurchlässig miteinander verbunden sind,
wobei der mindestens eine Schlauch (10, 14, 46, 54) zur Fluidzuführung nur mit einer der mindestens zwei Kappen (5, 6, 7, 8, 9) flüssigkeitsdurchlässig verbunden ist, wobei das pharmazeutische Fluid über die durch das eine Rohr (16, 18, 20) oder die zwei Rohre (16, 18, 20) miteinander verbundenen zumindest zwei Kappen (5, 6, 7, 8, 9) auf die zumindest zwei Knochenschrauben (1) verteilbar ist oder
wobei der mindestens eine Schlauch (10, 14, 46, 54) zur Fluidzuführung und ein Schlauch (44) zur Fluidableitung nur mit einer der mindestens zwei Kappen (5, 6, 7, 8, 9) flüssigkeitsdurchlässig verbunden sind, wobei das pharmazeutische Fluid über die durch das eine Rohr (16, 18, 20) oder die zwei Rohre (16, 18, 20) miteinander verbundenen zumindest zwei Kappen (5, 6, 7, 8, 9) seriell durch die zumindest zwei Knochenschrauben (1) leitbar ist.

13. Vorrichtung nach einem der vorangehenden Ansprüche, **dadurch gekennzeichnet, dass**
die Vorrichtung einen Behälter (57) für das pharmazeutische Fluid aufweist, wobei insbesondere in dem Behälter (57) ein pharmazeutisches Fluid enthalten ist, vorzugsweise enthaltend zumindest einen antibiotischen Wirkstoff, zumindest einen antimykotischen Wirkstoff und/oder zumindest ein Chemotherapeutikum, wobei bevorzugt der Behälter (57) einen Hohlzylinder mit einem axial im Hohlzylinder verschiebbaren Kolben (58) aufweist, der ein erstes Ende des Hohlzylinders verschließt, wobei der Hohlzylinder an einem dem ersten Ende gegenüberliegenden Ende eine Austragsöffnung aufweist, die mit dem mindestens einen Schlauch (10, 14, 46, 54) zur Fluidzuführung verbunden oder verbindbar ist, besonders bevorzugt über ein manuell bedienbares Ventilelement (59) zur Regulierung der Strömungsgeschwindigkeit des pharmazeutischen Fluids mit dem mindestens einen Schlauch (10, 14, 46, 54) zur Fluidzuführung verbunden oder verbindbar ist.

14. Vorrichtung nach einem der vorangehenden Ansprüche, **dadurch gekennzeichnet, dass**
die Vorrichtung eine Fördereinrichtung (56) aufweist, mit der das pharmazeutische Fluid aus einem mit der Fördereinrichtung (56) verbundenen oder verbindbaren Behälter (57) in den mindestens einen Schlauch (10, 14, 46, 54) zur Fluidzuführung, durch den Kanal (40) in der mindestens einen Kappe (5, 6, 7, 8, 9), durch den mindestens einen Kanal (38) im Schraubenkörper der mindestens einen Knochenschraube (1) und durch die wenigstens eine Fluidaustrittsöffnung (32) in die Umgebung der mindestens einen Knochenschraube (1) drückbar ist, wobei vorzugsweise
die Fördereinrichtung (56) ein Energiespeicherelement, insbesondere zumindest eine gespannte Feder (68), aufweist, wobei die Fördereinrichtung (56) mit Energie aus dem Energiespeicherelement antreibbar ist, wobei besonders bevorzugt mit dem Energiespeicherelement ein Kolben (58) in einem Hohlzylinder als der Behälter (57) in Richtung einer gegenüberliegenden Austragsöffnung anzutreiben ist.

15. Vorrichtung nach einem der vorangehenden Ansprüche, **dadurch gekennzeichnet, dass**
die Vorrichtung zumindest eine Verschlusskappe (22, 26) aufweist, mit der der mindestens eine Kanal (38) im Schraubenkörper der Knochenschraube (1) reversibel verschließbar ist, wobei vorzugsweise die zumindest eine Verschlusskappe (22, 26) einen proximalen Stift (48) aufweist, mit dem der mindestens eine Kanal (38) bis zur wenigstens einen Fluidaustrittsöffnung (32) reversibel verschließbar ist.

## Claims

1. A device for the local application of and/or for flushing with pharmaceutical fluids, said device having
at least one bone screw (1), said at least one bone screw (1) having a screw body, an outer thread (2) and a proximal screw head (3), wherein at least one conduit (38) is disposed in the screw body, wherein the at least one conduit (38) is fluid-permeable, begins at the screw head (3) and opens out into at least one fluid outlet opening (32) in the screw body, wherein the at least one fluid outlet opening (32) is spaced apart from the screw head (3) in the distal direction,
at least one axial groove (34) that penetrates the outer thread (2) of the bone screw (1), which extends from the at least one fluid outlet opening (32) in the screw body through to the screw head (3), wherein a base of the groove of the at least one axial groove (34) is deeper in the screw body as a base of the outer thread (2),
at least one cap (5, 6, 7, 8, 9) with a connecting element (39) for the detachable connection of the at least one cap (5, 6, 7, 8, 9) with the proximal screw head (3) of the at least one bone screw (1), wherein a fluid-permeable conduit (40) is arranged in the at least one cap (5, 6, 7, 8, 9), wherein said conduit (40) in the at least one cap (5, 6, 7, 8, 9) opens out into the at least one conduit (38) of the at least one bone screw (1), when the at least one cap (5, 6, 7, 8, 9) is detachably connected via the connecting element (39) to the at least one bone screw (1), and wherein the conduit (40) begins at an inlet opening in the at least one cap (5, 6, 7, 8, 9), **characterized in that** the device comprises at least one hose (10, 14, 46, 54) for feeding fluid, which is connected or connectable in a fluid-permeable manner with the inlet opening on one of the at least one cap (5, 6, 7, 8, 9), so that a pharmaceutical fluid is pressable from the at least one fluid outlet opening (32) with a pressure through the at least one hose (10, 14, 46, 54) for feeding fluid, through the conduit (40) of the at least one cap (5, 6, 7, 8, 9) and through the at least one conduit (38) of the at least one bone screw (1), when the at least one hose (10, 14, 46, 54) for feeding fluid is connected to the at least one cap (5, 6, 7, 8, 9) and the at least one cap (5, 6, 7, 8, 9) is connected via the connecting element (39) with the at least one bone screw (1).

2. The device according to Claim 1, **characterized in that**
the at least one bone screw (1) has at least one radial groove (36) which is disposed in the surface of the screw head (3) of the at least one bone screw (1) and which is connected to the at least one axial groove (34) in the screw body.

3. The device according to Claim 1 or 2, **characterized in that** the at least one axial groove (34) penetrating the outer thread (2) of the bone screw (1) extends from the at least on fluid outlet opening (32) on the distal screw body through to a distal side of the screw head (3), wherein, preferably, the at least one axial groove (34) extends through to the radial outer side of the screw head (3).

4. The device according to any one of the preceding claims, **characterized in that** the connecting element (39) is a protrusion on a lower side of the cap pointing to the screw head (3), wherein the protrusion comprises the conduit (40) that is fluid-permeable in the at least one cap (5, 6, 7, 8, 9), wherein the at least one cap (5, 6, 7, 8, 9) is reversibly inserted or insertable into the at least one conduit (38) of the at least one bone screw (1) with the protrusion, wherein, preferably, the protrusion is disposed in the center of the lower side of the cap, particularly preferably disposed axially along a symmetry axis of the at least one cap (5, 6, 7, 8, 9).

5. The device according to Claim 4, **characterized in that** the at least one cap (5, 6, 7, 8, 9) on the lower side of the cap pointing to the screw head (3) has a groove (42) that extends outwards from the protrusion in the radial direction, wherein the at least one groove (42) of the at least one cap (5, 6, 7, 8, 9) preferably extends up to the radial edge of the at least one cap (5, 6, 7, 8, 9) and/or a hose (44) is connected in a fluid-permeable manner to the at least one groove (42) of the at least one cap (5, 6, 7, 8, 9) for discharging fluid .

6. The device according to any one of the preceding claims, **characterized in that** the at least one hose (10, 14, 46, 54) for feeding fluid is connected or connectable to a fluid reservoir in a fluid-permeable manner, wherein a pharmaceutical fluid from the fluid reservoir is pressable under pressure into the at least one hose (10, 14, 46, 54) for feeding fluid through the conduit (40) of the at least one cap (5, 6, 7, 8, 9) and into the at least one conduit (38) of the at least one bone screw (1).

7. The device according to any one of the preceding claims, **characterized in that** aside from being connected on the at least one fluid outlet opening (32), the at least one conduit (38) in the screw body is not connected to the at least one axial groove (34) in the screw body.

8. The device according to any one of the preceding claims, **characterized in that** the at least one cap (5, 6, 7, 8, 9) is formed as a cupola with an underside that is planar with the exception of the connecting element (39) and/or the at least one cap (5, 6, 7, 8, 9) fully covers the screw head (3), preferably, the at least one cap (5, 6, 7, 8, 9) overlaps the screw head (3).

9. The device according to any one of the preceding claims, **characterized in that** the at least one cap (5, 6, 7, 8, 9) has one opening or multiple openings for discharging fluids, which are connectable or connected to a hose (44) for discharging fluid, wherein the opening or the openings is or are preferably disposed on a lower side of the cap of the at least one cap (5, 6, 7, 8, 9) pointing towards the screw head (3), and/or the at least one hose (46) for feeding fluid and the hose (44) for discharging fluid are interconnected in the longitudinal direction or are interconnected parallel adjacent to each other or are disposed coaxially in relation to each other.

10. The device according to any one of the preceding claims, **characterized in that** the device has at least one osteosynthesis plate (28), and/or
the at least one cap (5, 6, 7, 8, 9) has at least one latching element, with which the at least one cap (5, 6, 7, 8, 9) can be engaged with an osteosynthesis plate (28), wherein preferably, the at least one latching element is disposed on a lower side of the cap of the at least one cap (5, 6, 7, 8, 9) pointing towards the screw head (3).

11. The device according to any one of the preceding claims, **characterized in that** the at least one hose (10, 14, 46, 54) for feeding fluid has a maximum radial expansion of 5 percent, in particular, a maximum radial expansion of 1 percent, with an inner pressure of 5 bar, wherein preferably, a hose (44) for discharging fluid also has a maximum radial expansion of 5 percent, in particular, a maximum radial expansion of 1 percent, with an inner pressure of 5 bar.

12. The device according to any one of the preceding claims, **characterized in that** the at least one cap (5, 6, 7, 8, 9) is at least two caps (5, 6, 7, 8, 9), and the at least one bone screw (1) is at least two bone screws (1), wherein the at least two caps (5, 6, 7, 8, 9) are affixed with their connecting elements (39) in two different of the at least two bone screws (1), wherein the at least two caps (5, 6, 7, 8, 9) are interconnected via one tube (16, 18, 20) or two tubes (16, 18, 20) in a fluid-permeable manner, wherein the at least one hose (10, 14, 46, 54) for feeding fluid is only connected to one of the at least two caps (5, 6, 7, 8, 9), wherein the pharmaceutical fluid is distributable over the at least two bone screws (1) via the at least two caps (5, 6, 7, 8, 9) that are interconnected via the one tube (16, 18, 20) or the two tubes (16, 18, 20), or wherein the at least one hose (10, 14, 46, 54) for feeding fluid and a hose (44) for discharging fluid are only connected to one of the at least two caps (5, 6, 7, 8, 9) in a fluid-permeable manner, wherein the pharmaceutical fluid is serially directable through the at least two bone screws (1) via the at least two caps (5, 6, 7, 8, 9) that are interconnected via the one tube (16, 18, 20) or the two tubes (16, 18, 20).

13. The device according to any one of the preceding claims, **characterized in that** the device has a container (57) for the pharmaceutical fluid, wherein in particular a pharmaceutical fluid is contained in the container (57), preferably containing at least one antibiotic active ingredient, at least one antimycotic active ingredient and/or at least one chemotherapeutic ingredient, wherein preferably, the container (57) is a hollow cylinder with a piston (58) that is axially movable in the hollow cylinder, which closes a first end of the hollow cylinder, wherein the hollow cylinder has a delivery opening on an end positioned opposite the first end, which is connected or connectable to the at least one hose (10, 14, 46, 54) for the fluid feed, preferably, is connected or connectable to the at least one hose (10, 14, 46, 54) via a manually operable valve element (59) for regulating the flow speed of the pharmaceutical fluid.

14. The device according to any one of the preceding claims, **characterized in that** The device has a conveyor apparatus (56), with which the pharmaceutical fluid is pressable out of a container (57) that is connected or connectable to the conveyor apparatus (56), into the at least one hose (10, 14, 46, 54) for the fluid feed, through the conduit (40) in the at least one cap (5, 6, 7, 8, 9), through the at least one conduit (38) in the screw body of the at least one bone screw (1) and through the at least one fluid outlet opening (32) into the surrounding area of the at least one bone screw (1), wherein preferably,
the conveyor apparatus (56) has an energy storage element, in particular at least one tensioned spring (68), wherein the conveyor apparatus (56) is drivable with energy from the energy storage element, wherein preferably, with the energy storage element, a piston (58) in a hollow cylinder is to be driven as the container (57) in the direction of an opposite delivery opening.

15. The device according to any one of the preceding claims, **characterized in that** the device has at least one locking cap (22, 26), by which the at least one conduit (38) is reversibly lockable in the screw body of the bone screw (1), wherein preferably, the at least one locking cap (22, 26) has a proximal pin (48) with which the at least one conduit (38) is reversibly lockable up to at least one fluid outlet opening (32).

## Revendications

1. Dispositif d'application locale de fluides pharmaceutiques et/ou de rinçage par des fluides pharmaceutiques, le dispositif présentant
au moins une vis pour ostéosynthèse (1), ladite au moins une vis pour ostéosynthèse (1) présentant un corps de vis, une filetage extérieur (2) et une tête de vis (3) proximale, au moins un canal (38) étant disposé dans le corps de vis, ledit au moins un canal (38) étant perméable aux liquides, partant de la tête de vis (3) et débouchant dans au moins une ouverture de sortie de fluide (32) dans le corps de vis, ladite au moins une ouverture de sortie de fluide (32) étant écartée de la tête de vis (3) dans la direction distale,
au moins une rainure axiale (34) traversant le filetage extérieur (2) de la vis pour ostéosynthèse (1), laquelle rainure s'étend depuis ladite au moins une ouverture de sortie de fluide (32) dans le corps de vis jusqu'à la tête de vis (3), un fond de rainure de ladite au moins une rainure axiale (34) dans le corps de vis étant plus profond qu'un fond du filetage extérieur (2),
au moins un capuchon (5, 6, 7, 8, 9) comprenant un élément de liaison (39) pour la liaison démontable dudit au moins un capuchon (5, 6, 7, 8, 9) à la tête de vis (3) proximale de ladite au moins une vis pour ostéosynthèse (1), un canal (40) perméable aux liquides étant disposé dans ledit au moins un capuchon (5, 6, 7, 8, 9), le canal (40) dans ledit au moins un capuchon (5, 6, 7, 8, 9) débouchant dans ledit au moins un canal (38) de ladite au moins une vis pour ostéosynthèse (1), lorsque ledit au moins un capuchon (5, 6, 7, 8, 9) est relié de manière démontable par l'intermédiaire de l'élément de liaison (39) à ladite au moins une vis pour ostéosynthèse (1) et le canal (40) commençant au niveau d'une ouverture d'entrée dans ledit au moins un capuchon (5, 6, 7, 8, 9), **caractérisé en ce que** le dispositif présente au moins un tuyau (10, 14, 46, 54) pour l'alimentation en fluide, lequel tuyau est ou peut être relié de manière perméable aux liquides à l'ouverture d'entrée sur un dudit au moins un capuchon (5, 6, 7, 8, 9), de sorte qu'un fluide pharmaceutique peut être pressé sous une pression à travers ledit au moins un tuyau (10, 14, 46, 54) pour l'alimentation en fluide, à travers le canal (40) dudit au moins un capuchon (5, 6, 7, 8, 9) et à travers ledit au moins un canal (38) de ladite au moins une vis pour ostéosynthèse (1) hors de ladite au moins une ouverture de sortie de fluide (32), lorsque ledit au moins un tuyau (10, 14, 46, 54) pour l'alimentation en fluide est relié audit au moins un capuchon (5, 6, 7, 8, 9) et ledit au moins un capuchon (5, 6, 7, 8, 9) est relié par l'intermédiaire de l'élément de liaison (39) à ladite au moins une vis pour ostéosynthèse (1).

2. Dispositif selon la revendication 1, **caractérisé en ce que**
ladite au moins une vis pour ostéosynthèse (1) présente au moins une rainure radiale (36), laquelle rainure est disposée dans la surface de la tête de vis (3) de ladite au moins une vis pour ostéosynthèse (1) et laquelle rainure est reliée à ladite au moins une rainure axiale (34) dans le corps de vis.

3. Dispositif selon la revendication 1 ou 2, **caractérisé en ce que**
ladite au moins une rainure axiale (34) traversant le filetage extérieur (2) de la vis pour ostéosynthèse (1) s'étend depuis ladite au moins une ouverture de sortie de fluide (32) au niveau du corps de vis distal jusqu'à un côté distal de la tête de vis (3), ladite au moins une rainure axiale (34) s'étendant de préférence jusqu'au côté de la tête de vis (3) situé radialement à l'extérieur.

4. Dispositif selon l'une quelconque des revendications précédentes, **caractérisé en ce que** l'élément de liaison (39) est une saillie sur une face inférieure du capuchon faisant face à la tête de vis (3), la saillie comprenant le canal (40) perméable aux liquides dans ledit au moins un capuchon (5, 6, 7, 8, 9). ledit au moins un capuchon (5, 6, 7, 8, 9) étant ou pouvant être inséré de manière réversible par la saillie dans ledit au moins un canal (38) de ladite au moins une vis pour ostéosynthèse (1), la saillie étant de préférence disposée au centre de la face inférieure du capuchon, étant de manière particulièrement préférée disposée axialement le long d'un axe de symétrie dudit au moins un capuchon (5, 6, 7, 8, 9).

5. Dispositif selon la revendication 4, **caractérisé en ce que**
ledit au moins un capuchon (5, 6, 7, 8, 9) présente sur la face inférieure de capuchon faisant face à la tête de vis (3) au moins une rainure (42) s'étendant dans la direction radiale depuis la saillie vers l'extérieur, ladite au moins une rainure (42) dudit au moins un capuchon (5, 6, 7, 8, 9) s'étendant de préférence jusqu'au bord radial dudit au moins un capuchon (5, 6, 7, 8, 9) et/ou un tuyau (44) pour l'évacuation de fluide étant relié de manière perméable aux liquides à ladite au moins une rainure (42) dudit au moins un capuchon (5, 6, 7, 8, 9).

6. Dispositif selon l'une quelconque des revendications précédentes, **caractérisé en ce que** ledit au moins un tuyau (10, 14, 46, 54) pour l'alimentation en fluide est ou peut être relié de manière perméable aux liquides à un réservoir de fluide, un fluide pharmaceutique pouvant être pressé sous pression depuis le réservoir de fluide dans ledit au moins un tuyau (10, 14, 46, 54) pour l'alimentation en fluide à travers le canal (40) dudit au moins un capuchon (5, 6, 7, 8, 9) et à travers ledit au moins un canal (38) de ladite au moins une vis pour ostéosynthèse (1).

7. Dispositif selon l'une quelconque des revendications précédentes, **caractérisé en ce que** ledit au moins un canal (38) dans le corps de vis n'est pas relié à ladite au moins une rainure axiale (34) dans le corps de vis excepté au niveau de ladite au moins une ouverture de sortie de fluide (32).

8. Dispositif selon l'une quelconque des revendications précédentes, **caractérisé en ce que** ledit au moins un capuchon (5, 6, 7, 8, 9) est conçu en forme de dôme avec une face inférieure plane, excepté l'élément de liaison (39), et/ou ledit au moins un capuchon (5, 6, 7, 8, 9) couvre complètement la tête de vis (3), de préférence ledit au moins un capuchon (5, 6, 7, 8, 9) chevauche la tête de vis (3).

9. Dispositif selon l'une quelconque des revendications précédentes, **caractérisé en ce que** ledit au moins un capuchon (5, 6, 7, 8, 9) présente une ouverture ou plusieurs ouvertures pour l'évacuation de fluides, qui est/sont ou peut/peuvent être reliée(s) à un tuyau (44) pour l'évacuation de fluides, l'ouverture ou les ouvertures étant de préférence disposée(s) au niveau d'une face inférieure de capuchon dudit au moins un capuchon (5, 6, 7, 8, 9) faisant face à la tête de vis (3) et/ou
ledit au moins un tuyau (46) pour l'alimentation en fluide et le tuyau (44) pour l'évacuation de fluides étant reliés l'un à l'autre dans la direction longitudinale, de préférence reliés l'un à l'autre parallèlement l'un à côté de l'autre ou disposés coaxialement l'un par rapport à l'autre.

10. Dispositif selon l'une quelconque des revendications précédentes, **caractérisé en ce que** le dispositif présente au moins une plaque d'ostéosynthèse (28) et/ou ledit au moins un capuchon (5, 6, 7, 8, 9) présente au moins un élément d'encliquetage, au moyen duquel ledit au moins un capuchon (5, 6, 7, 8, 9) doit être encliqueté avec une plaque d'ostéosynthèse (28), ledit au moins un élément d'encliquetage étant disposé de préférence au niveau d'une face inférieure de capuchon dudit au moins un capuchon (5, 6, 7, 8, 9) faisant face à la tête de vis (3).

11. Dispositif selon l'une quelconque des revendications précédentes, **caractérisé en ce que** ledit au moins un tuyau (10, 14, 46, 54) pour l'alimentation en fluide présente une dilatation radiale maximale de 5 pour cent, en particulier une dilatation radiale maximale de 1 pour cent, à une pression interne de 5 bars, un tuyau (44) pour l'évacuation de fluides présentant de préférence aussi une dilatation radiale maximale de 5 pour cent, en particulier une dilatation radiale maximale de 1 pour cent, pour une pression interne de 5 bars.

12. Dispositif selon l'une quelconque des revendications précédentes, **caractérisé en ce que** ledit au moins un capuchon (5, 6, 7, 8, 9) est constitué par au moins deux capuchons (5, 6, 7, 8, 9) et ladite au moins une vis pour ostéosynthèse (1) est constituée par au moins deux vis pour ostéosynthèse (1), lesdits au moins deux capuchons (5, 6, 7, 8, 9) étant fixés au moyen de leur éléments de liaison (39) dans deux vis différentes parmi lesdites au moins deux vis pour ostéosynthèse (1), lesdits au moins deux capuchons (5, 6, 7, 8, 9) étant reliés l'un à l'autre de manière perméable aux liquides au moyen d'un tube (16, 18, 20) ou de deux tubes (16, 18, 20),
ledit au moins un tuyau (10, 14, 46, 54) pour l'alimentation en fluide étant relié de manière perméable aux liquides à seulement un desdits au moins deux capuchons (5, 6, 7, 8, 9), le fluide pharmaceutique pouvant être réparti sur lesdites au moins deux vis pour ostéosynthèse (1) par l'intermédiaire desdits au moins deux capuchons (5, 6, 7, 8, 9) reliés l'un à l'autre au moyen dudit un tube (16, 18, 20) ou desdits deux tubes (16, 18, 20) ou
ledit au moins un tuyau (10, 14, 46, 54) pour l'alimentation en fluide et un tuyau (44) pour l'évacuation de fluide étant reliés de manière perméable aux liquides à seulement l'un desdits au moins deux capuchons (5, 6, 7, 8, 9), le fluide pharmaceutique pouvant être guidé en série à travers lesdites au moins deux vis pour ostéosynthèse (1) par l'intermédiaire desdits au moins deux capuchons (5, 6, 7, 8, 9) reliés l'un à l'autre au moyen dudit un tube (16, 18, 20) ou desdits deux tubes (16, 18, 20).

13. Dispositif selon l'une quelconque des revendications précédentes, **caractérisé en ce que** le dispositif présente un récipient (57) pour le fluide pharmaceutique, en particulier un fluide pharmaceutique étant contenu dans le récipient (57), contenant de préférence au moins un agent antibiotique, au moins un agent antifongique et/ou au moins un chimiothérapeutique, le récipient (57) présentant de préférence un cylindre creux comprenant un piston (58) pouvant être déplacé axialement dans le cylindre creux, lequel piston ferme une première extrémité du cylindre creux, le cylindre creux présentant, en une extrémité opposée à la première extrémité, une ouverture de décharge qui est ou peut être reliée audit au moins un tuyau (10, 14, 46, 54) pour l'alimentation en fluide, qui est ou peut être reliée de manière particulièrement préférée audit au moins un tuyau (10, 14, 46, 54) pour l'alimentation en fluide par l'intermédiaire d'un élément de soupape (59), qui peut être commandé manuellement pour la régulation de la vitesse d'écoulement du fluide pharmaceutique.

14. Dispositif selon l'une quelconque des revendications précédentes, **caractérisé en ce que** le dispositif présente un dispositif de transport (56), au moyen duquel le fluide pharmaceutique peut être pressé hors d'un récipient (57) relié ou pouvant être relié au dispositif de transport (56) dans ledit au moins un tuyau (10, 14, 46, 54) pour l'alimentation en fluide, à travers le canal (40) dans ledit au moins un capuchon (5, 6, 7, 8, 9), à travers ledit au moins un canal (38) dans le corps de vis de ladite au moins une vis pour ostéosynthèse (1) et à travers ladite au moins une ouverture de sortie de fluide (32) dans l'environnement de ladite au moins une vis pour ostéosynthèse (1),
le dispositif de transport (56) présentant de préférence un élément de stockage d'énergie, en particulier au moins un ressort (68) tendu, le dispositif de transport (56) pouvant être entraîné au moyen d'énergie de l'élément de stockage d'énergie, un piston (58) dans un cylindre creux en tant que le récipient (57) pouvant entre entraîné de manière particulièrement préférée au moyen de l'élément de stockage d'énergie dans la direction d'une ouverture de décharge opposée.

15. Dispositif selon l'une quelconque des revendications précédentes, **caractérisé en ce que** le dispositif présente au moins un capuchon de fermeture (22, 26), au moyen duquel ledit au moins un canal (38) dans le corps de vis de la vis pour ostéosynthèse (1) peut être fermé de manière réversible, ledit au moins un capuchon de fermeture (22, 26) présentant de préférence une tige (48) proximale, au moyen de laquelle ledit au moins un canal (38) peut être fermé de manière réversible jusqu'à ladite au moins une ouverture de sortie de fluide (32).
